# EUROPEAN PATENT APPLICATION

(11) **EP 2 594 290 A1**
(43) Date of publication of application: **22.05.2013**
(21) Application number: 11189740.1
(22) Date of filing: 18.11.2011
(51) Int. Cl.: A61K 47/48, A61K 51/08, C07K 5/02, C07K 5/06, C07K 5/08, A61K 49/00

(54) **Selective imaging of actin in live native cells**

(71) Applicant: Max-Planck-Gesellschaft zur Förderung der Wissenschaften e.V., 80539 München (DE); Technische Universität Dortmund, 44227 Dortmund (DE)
(72) Inventor: The designation of the inventor has not yet been filed
(74) Representative: Heubeck, Christian

(57) **Abstract**

The present invention provides cyclodepsipeptidic compounds, which selectively bind to actin structures, their manufacture and their use as probes for imaging and targeting actin structures, particularly in living cells.

## Description

The present invention refers to cyclodepsipeptidic compounds, which selectively bind to actin structures, their manufacture and their use as probes for imaging and targeting actin structures, particularly in living cells.

The tightly regulated, dynamic equilibrium between monomeric G-actin and helical-filamentous F-actin structures is a key component of important cellular functions in normal and pathological states, and impacts cell motility, endocytosis, and cytokinesis (Disanza et al. Cell. Mol. Life Sci. 62, 955-970, 2005; Schmidt et al. Rev. Cell Dev. Biol. 1998, 14, 305-338). Means to image actin have become indispensable for studying actin and its interaction with cell components, prominently by fluorescence-based microscopy (Small et al. Microsc. Res. Tech. 1999, 47, 3-17; Rodriguez et al. Nat. Cell Biol. 2003, 5, 599-609, Bugyi et al. Annu. Rev. Biophys. 2010, 9, 449-70). Conversely, attachment of fluorescent reporters to small molecule probes has been applied very successfully for target elucidation studies by using comparative cell microscopy or high-content screening (Abraham et al. Trends Biotechnol. 2004, 22, 15-22; Bleicher et al., Nat. Rev. Drug Discov. 2003, 2, 369-378; Sumiya et al. ACS Chem Biol. 2011, 6, 425-431; Matus et al. Proc. Natl. Acad. Sci. U. S. A. 1982, 79, 7590-7594).

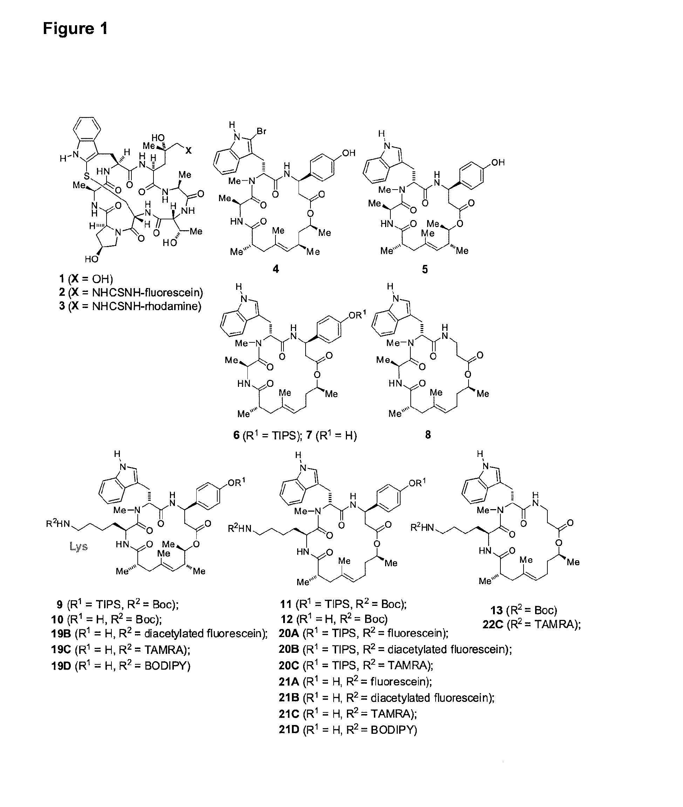

In fixed, permeabilized cells, typical actin structures can be imaged *post mortem* by using fluorescent labels either on anti-actin antibodies or on phalloidins, small molecule heptapeptide toxins from the death cap mushrooms *Amanita sp.* Labeled phalloidins (Figure 1, **1**) have helped to shape our current knowledge about actin cell biology (Matus et al.; Kuczmarski et al. J. Cell. Biol. 1979, 80, 356-371; Ab-El-Basset et al. J. Chem. Neuroanat. 2, 7, 113-122; Wulf et al Proc. Natl. Acad. Sci. U. S. A. 1979, 71, 4498-4502; Wieland, Science 1968, 159, 946-952; Lengsfeld et al. Proc. Natl. Acad. Sci. U. S. A. 1974, 71, 2803-2807). Today, semi-synthetic fluorescent conjugates of phalloidin (Figure 1, e.g. **2-3**) are widely used. They selectively bind to the F-actin quaternary structure and therefore reach excellent s/n ratios, but cannot visualize other actin pools.

In live cells, actin structures can be observed either by microinjecting fluorescently labeled actin proteins into living cells or by co-expressing fusions of fluorescent protein with actin, such as GFP-actin (Choidas et al. Eur. J. Cell Biol. 1998, 77, 81-90; Westphal et al. Curr. Biol. 1997, 7, 176-183). DNA vectors for co-expressing fluorescent (GFP-, Cherry-) actin fusion proteins are routinely applied for transfection of human cell lines. However, for primary cells, tissues, or for many non-mammalian cells, transfection is often difficult or unreliable. Moreover, overexpression of GFP-tagged actin is known to interfere with cytoskeletal dynamics, causing aberrations in myosin processivity and nucleogenesis (Westphal et al.). F-actin binding protein tags were introduced as less disturbing technology, but still are not independent of manipulating the cells (Riedl et al. Nat. Methods 2008, 5, 605-607; Pang et al. Curr. Biology 1998, 8, 405-408). Thus, more versatile probes could be extremely useful for unrestricted monitoring of actin structures in native cells.

Similar to phalloidin (**1**), the cyclodepsipeptide natural products jasplakinolide (or 'jaspamide', **4**) and chondramide C (**5**, Figure 1) were found to stabilize F-actin, resulting in potent cytotoxicity (Crews et al. Tetrahedron Lett. 1986, 27, 2797-2800; Crews et al. J. Org. Chem. 1994, 59, 2932-2934; Talpir et al. Tetrahedron Lett. 1994, 35, 4453-4456; Zabriskie et al. J. Am. Chem. Soc. 1986, 108, 3123-3124; Chevallier et al. Org. Lett. 2003, 5, 3737-3739). In contrast to phalloidin, **4** and **5** easily penetrate cells and were considered for drug applications, but not developed further (Senderowicz et al. J. Natl. Cancer I. 1995, 87, 46-51; Stingl et al. Cancer Chemother. Pharmacol. 1992, 30, 401-406; Odaka et al. Clin. Diagn. Lab. Immunol. 2000, 7, 947-952; Newman et al. Curr. Cancer Drug Targets 2002, 2, 279-308). Compared to phalloidin, compounds **4** and **5** can be effectively prepared by total synthesis (Schuresko et al. Angew. Chem. Int. Ed. 2007, 46, 3547-3549; Tannert et al. J. Am. Chem. Soc. 2010, 132, 3063-3077; Waldmann et al. Angew. Chem., Int. Ed. 2008, 47, 6473-6477). Moreover, by targeted structural variation, high acute toxicity might be reduced to increase applicability in live cell studies. Most importantly, such probes should have a high propensity to enter living cells, potentially providing generic actin targeting probes for cell microscopy.

Thus, the object of the present invention is the provision of easily accessible compounds, which selectively bind to actin structures. Moreover, reduced cytotoxicity, improved propensity to enter living cells or/and reduced interference with cytoskeletal dynamics in living cells are desired to provide compounds suitable as probes, particularly in living cells.

The object of the present invention is solved by compounds according to formula (I)

### Summary of the Invention

Thus, in a first aspect, the invention is directed to compounds according to Formula (I) 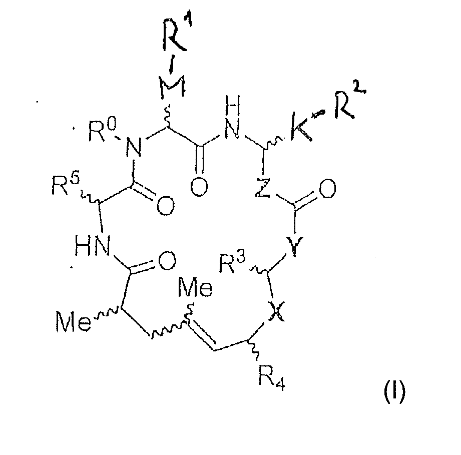 wherein
R⁰ is hydrogen, hydroxy, substituted or unsubstituted C₁₋₆-alkyl, substituted or unsubstituted hydroxy-C₁₋₆-alkyl, substituted or unsubstituted hydroxy-C₃₋₈-cycloalkyl or a substituted or unsubstituted 3-14 membered carbocyclic ring comprising 0-2 heteroatoms selected from the group consisting of N, O or S, M is a bond or -(CH₂)ₙ- with n being an integer of 1-4, preferably -(CH₂)-,
K is a bond or -(CH₂)ₙ-, preferably a bond,
R¹ and R² idependently are hydrogen, halogen, a 3-14 membered carbocylic ring comprising 0-2 heteroatoms selected from the group consisting of N, O or S optionally substituted by at least one of halogen, -R⁶, -OR⁶, -SR⁶, -N(R⁶)₂, -NO₂ wherein R⁶ independently is hydrogen, substituted or unsubstituted C₁₋₆-alkyl, substituted or unsubstituted C₃₋₈-cycloalkyl or a protective group,
R³, R⁴ independently are hydrogen, unsubstituted or substituted C₁-C₆-alkyl, R⁵ is -L-CG, wherein -L- is a spacer or a bond and -CG is an optionally protected coupling group,
X and Z independently are a bond or -CHR⁷-, wherein R⁷ is hydrogen or unsubstituted or substituted C₁-C₆-alkyl,
Y is -CR⁹R¹⁰-, -NR⁹-, -N(OR⁹)-, -N(NR⁹R¹⁰)-, -NR⁹NR¹⁰-, or -O-, particularly -O-, and
R⁹, R¹⁰ independently are hydrogen, hydroxy, amino, unsubstituted or substituted C₁₋₆-alkyl, or unsubstituted or substituted C₃₋₈-cycloalkyl.

As indicated in the structure of Formula (I), the compounds encompass any configuration at any of the asymmetric carbon atoms, i.e. (S)- or (R)-configuration. Moreover, the C=C double bond in Formula (I) furthermore may be E- or Z-configured and is preferably E-configured. In a preferred embodiment of the invention, the compound has the Formula (II).

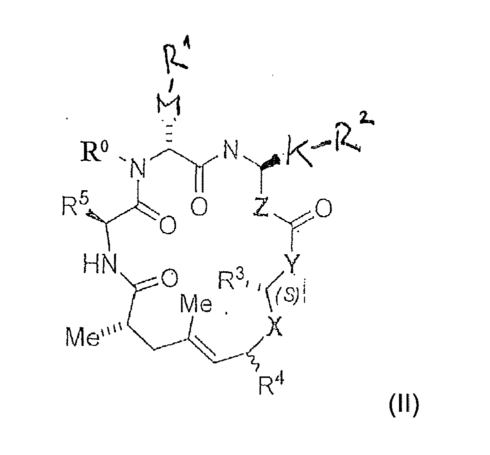

According to the present invention the term "*substituted*" means preferably at least one of a substituent selected from the group consisting of halogen up to per-halo, cyano, -R⁶, -OR⁶, -SR⁶, -N(R⁶)₂, -NO₂, -COR⁶, -CON(R⁶)₂, -COOR⁶, -OCOR⁶ and oxo, more preferably halogen, -R⁶, -OR⁶, -SR⁶, -N(R⁶)₂, and -NO₂.

According to the present invention, the term "*C₁₋₆-alkyl*" refers preferably to an alkyl group having 1-6, preferably 1-4, carbon atoms, such as methyl, ethyl, propyl, i-butyl, n-butyl, preferably methyl, ethyl and propyl, and more preferably methyl.

The term "*hydroxy-C₁₋₆-alkyl*" means preferably a C₁₋₆-alkyl group as defined above having at least one hydroxy substituent.

"*C₃₋₈-cycloalkyl*" according to the present invention may be a saturated carbocyclic ring structure, such as cyclopropane, cyclobutane, cyclopentane, cyclohexane or cyclooctane, preferably cyclopentane or cyclohexane. Accordingly, the term "*hydroxy-C₃₋₈-cycloalkyl"* relates to a cycloalkyl group as described above having at least one hydroxy substituent.

The term *"3-14 membered carbocyclic ring comprising 0-2 heteroatoms selected from the group consisting of N,* O *or S*" means preferably saturated, partially saturated or unsaturated cyclic rings, having 3-14 carbon atoms, one or two of which may be replaced by heteroatoms, such as N, O or S.

Examples for saturated carbocyclic rings are particularly C₃₋₈-cycloalkyl, such as cyclopropyl, cyclopentyl, cyclohexyl, and cyclooctyl, and C₃₋₈-heterocyclyl, such as tetrahydrofuranyl, thiolanyl, pyrrolidinyl, oxanyl, piperidinyl, dioxanyl, dithianyl, morpholinyl, or piperazinyl.

Partially saturated cyclic rings are particularly partially saturated heterocyclic rings, e.g. dihydrofuranyl, dihydrothiophenyl, dihydropyrroloyl, dihydropyranyl, preferably dihydrofuranyl.

Unsaturated carbocyclic rings are preferably aromatic or heteroaromatic rings. Preferred aromatic rings are phenyl or naphtyl, preferably phenyl. Preferred heteroaromatic rings are furanyl, thiophenyl, pyrrolyl, isoxazolyl, oxazolyl, isothiazolyl, thiazolyl, pyrazolyl, imidazolyl, pyridinyl, pyridazinyl, pyrimidinyl, pyrazinyl, indolyl, quinolinyl, isoquinolinyl or naphthyridinyl, preferably indolyl, more preferably indolyl.

In a preferred embodiment *"3-14 membered carbocyclic ring comprising 0-2 heteroatoms selected from the group consisting of N, O or S*" is a 6-14-membered aromatic or heteroaromatic ring comprising 0-2 heteroatoms selected from the group consisting of N, O or S, preferably phenyl, indolyl, naphthyl, more preferably phenyl or indolyl.

The term "halogen" refers to F, Cl, Br, I, preferably Br and Cl, more preferably Cl.

The term "protective group" refers preferably to substituents which are introduced into a molecule to protect a specific functional group temporarily and thus to prevent an undesired reaction. After the desired reaction has been carried out the protective group may be cleaved off under certain and known conditions. The technique applying protective groups is well-known to a person skilled in the art (Wutz and Greene, Greene's Protective Groups in Organic Synthesis, 4th ed., 2006, Wiley, incorporated herein by reference). Preferred protective groups are TIPS, Boc, Fmoc, Cbz, Ts, Alloc, preferably TIPPS, Boc, Fmoc, Cbz, Ts or Alloc.

The term "coupling group" refers preferably to groups capable of reacting with another functional group, thereby forming a covalent bond. Examples for coupling groups are halogens, amines such as -NHR⁸, -OR⁸, halogen, isocyanate, cyanide, -COCI, hydroxy, preferably -NHR⁸ or -OR⁸.

In a preferred embodiment of the present invention R⁰ is a subsituted or unsubstituted C₁-C₆-alkyl, particularly methyl, ethyl, or propyl, more preferably methyl.

In a preferred embodiment of the present invention R¹ is a substituted or unsubstituted 3-14 membered carbocyclic ring comprising 0-2 heteroatoms, more preferably 3-14-, even more preferably 6-14-, membered aromatic or heteroaromatic ring comprising 0-2 heteroatoms selected from the group consisting of N, O or S. In a more preferred embodiment R¹ is a 6-14 membered heteroaromatic ring, such as indolyl, more preferably 3-indolyl. Each of the carbocyclic rings may be substituted by at least one of halogen, -R⁶, -OR⁶, -SR⁶., -N(R⁶)₂, -NO₂. In one preferred embodiment, R¹ is unsubstituted 3-indolyl. In another preferred embodiment 3-indolyl is substituted in 1-position, preferably by C₁-₆-alkyl, more preferably by methyl.

In a preferred embodiment, M is -(CH₂)ₙ- with n being an integer of 1-4, preferably 1.

In one aspect of the invention R² is hydrogen

In another aspect of the invention R² is a substituted or unsubstituted 3-14 membered carbocyclic ring comprising 0-2 heteroatoms, more preferably 3-14-, even more preferably 6-14-, membered aromatic or heteroaromatic ring comprising 0-2 heteroatoms selected from the group consisting of N, O or S. In a more preferred embodiment R² is a 3-14 membered aromatic ring, such as phenyl or naphthyl, more preferably phenyl. Each of the carbocyclic rings may be substituted by at least one of halogen, -R⁶, -OR⁶, -SR⁶., -N(R⁶)_{2,} -NO₂ preferably -OR⁶, wherein R⁶ is preferably hydrogen or a protective group, such as TIPS, more preferably hydrogen. In one preferred embodiment, R² is at least substituted in para-position. In the most preferred embodiment of the invention R² is phenyl substituted by -OR⁶ in para-position, wherein R⁶ is hydrogen or TIPS.

In another embodiment, K is -(CH₂)ₙ- with n being an integer of 1-4, preferably 1.

In one embodiment R³ may be hydrogen. In another preferred embodiment R³ represents substituted or unsubstituted C₁-C₆-alkyl, such as methyl, ethyl, propyl, i-butyl, n-butyl, preferably methyl. The alkyl group is preferably unsubstituted. In another embodiment the alkyl group may be substituted by at least one of halogen up to per-halo, -R⁶, -OR⁶, -SR⁶, N(R⁶)₂, -NO₂ preferably by halogen up to per-halo.

In one embodiment R⁴ may be hydrogen. In another preferred embodiment R⁴ represents substituted or unsubstituted C₁-C₆-alkyl, such as methyl, ethyl, propyl, i-butyl, n-butyl, preferably methyl. The alkyl group is preferably unsubstituted. In another embodiment the alkyl group may be substituted by at least one of halogen up to per-halo, -R⁶, -OR⁶, -SR⁶, -N(R⁶)_{2,} -NO₂ preferably by halogen up to per-halo.

R⁶ is preferably hydrogen or a protective group such as TIPS.

Substituent X may be -CHR⁷-, wherein R⁷ is hydrogen or unsubstituted or substituted C₁-₆-alkyl, such as methyl, ethyl, or propyl, preferably methyl. In a preferred embodiment R⁷ is unsubstituted C₁-₆-alkyl. In another preferred embodiment R⁷ is hydrogen.

In another embodiment of the invention X is bond.

Z may be -CHR⁷- with R⁷ being as defined above. Preferably, Z represents -CH₂-. In another aspect of the invention Z is a bond.

L may be selected from the group consisting of -(CH₂)ₘ-, -(CH₂CH₂O)ₘ-, -(CH₂)ₘ-NHC(O)-(CH₂)ₘ-, -(CH₂)ₘ-OC(O)-(CH₂)ₘ-, particularly -(CH₂)ₘ- or -(CH₂)ₘ-NHC(O)-(CH₂)ₘ-, wherein m independently is an integer of 0-6, particularly 1-6, particularly 2-4, more particularly 4 or 5.

The coupling group CG is preferably -NHR⁸ or -OR⁸, particularly NHR⁸. In one embodiment R⁸ is preferably hydrogen or a protective group such as Boc, Fmoc, Cbz, Ts, Alloc, preferably Boc. In a preferred embodiment R⁸ is a protective group.

In a preferred embodiment R⁵ is -(CH₂)₄-NH-Boc.

In another embodiment R⁸ may be a diagnostic and/or therapeutic agent, particularly a diagnostic agent, or derivatives thereof. The diagnostic agent and/or therapeutic agent is covalently coupled at the coupling group via an amide, ester, ether, thioether, amine, carbonate, urea or a semicarbazide linkage, preferably via an amide linkage.

The diagnostic agent may be selected from the group consisting of a radionuclide, dye such as fluorescent dye, a spin label, a metal chelating agent, quantum dots, chromogens or co-factors such as luziferines, a protein-binding group such as biotin, a protein-reactive group such as a Michael acceptor, a photoreactive group such as benzophenone, or an antigenic moiety.

Preferably, the diagnostic agent is a fluorescent dye such as fluorescein, diacetylated fluorescein, TAMRA, BODIPY, rhodamine, coumarine, cyanine or derivatives thereof, particularly BODIPY or TAMRA, more particularly BODIPY. Dye derivatives according to the present invention exhibit a functional group, such as -COOH, or -COCI, particularly -COOH, which can optionally be bound to a spacer as defined for substituent R⁵ above. Preferred derivatives are BODIPY-FL-propionic acid.

The therapeutic agent may be selected from a toxin, a drug, a signalling molecule or hormone, or a peptide. The drug may be a cytotoxic or cytostatic agent, an antirheumatic agent, an enzyme inhibitor or enzyme activator.

Preferred compounds according to the present invention have a structure according to Formulae (IV), (V), (VI) or (VII).

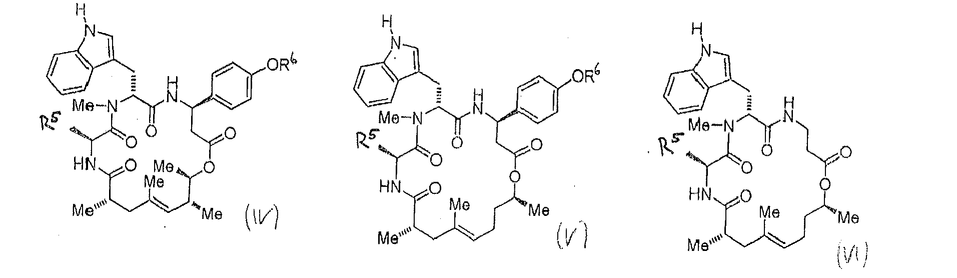

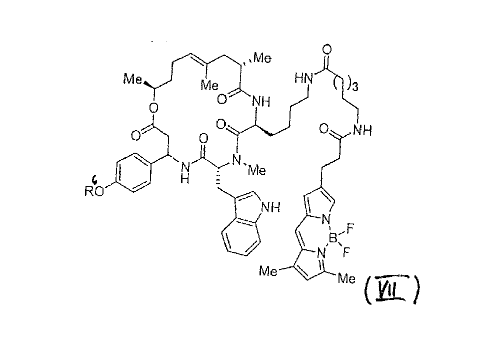

In another aspect, the present invention relates to a process for the manufacture of a compound according to the invention, comprising the steps:
(i) providing a compound according to Formula (III) 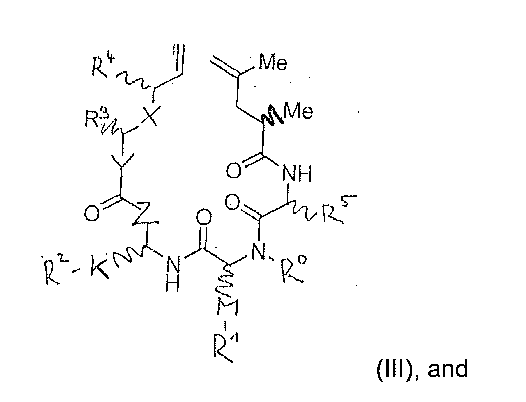
(ii) converting the compound of Formula (III) to a compound of Formula (I) using a metathesis catalyst.

The starting material of Formula (III) may be produced by a 5-step synthesis as shown in Scheme 1.

Suitable metathesis catalysts are metal carbene complexes, wherein the metal atom, such as molybdenum, tungsten, ruthenium or tantal, is preferably in a high oxidation state. Particularly, Grubbs- or Schrock-type catalysts known in the art can be applied with good results (Yu et al., Nature 2011, 479, 88-93; Nolan et al., Chem. Soc. Rev. 2010, 39, 3305-3316; M. Bieniek et al. Chem.-Eur. J., 2008, 14, 806-818). In a preferred embodiment the metathesis catalysts are selected from the group consisting of catalysts C1 - C3.

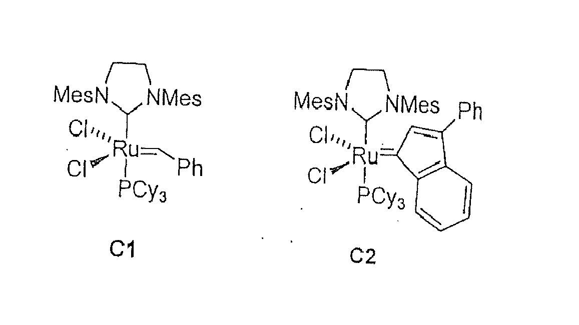

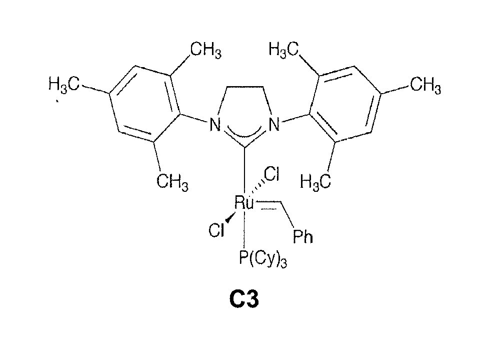

The metathesis reaction is preferably carried out in organic solvents, e.g., dichloromethane, at temperatures from 20-60°C, preferably 30-50°C, more preferably 35-45°C over a period of 1-10 h, preferably 2-6 h. The respective reaction conditions depend on selected catalysts and may therefore vary. 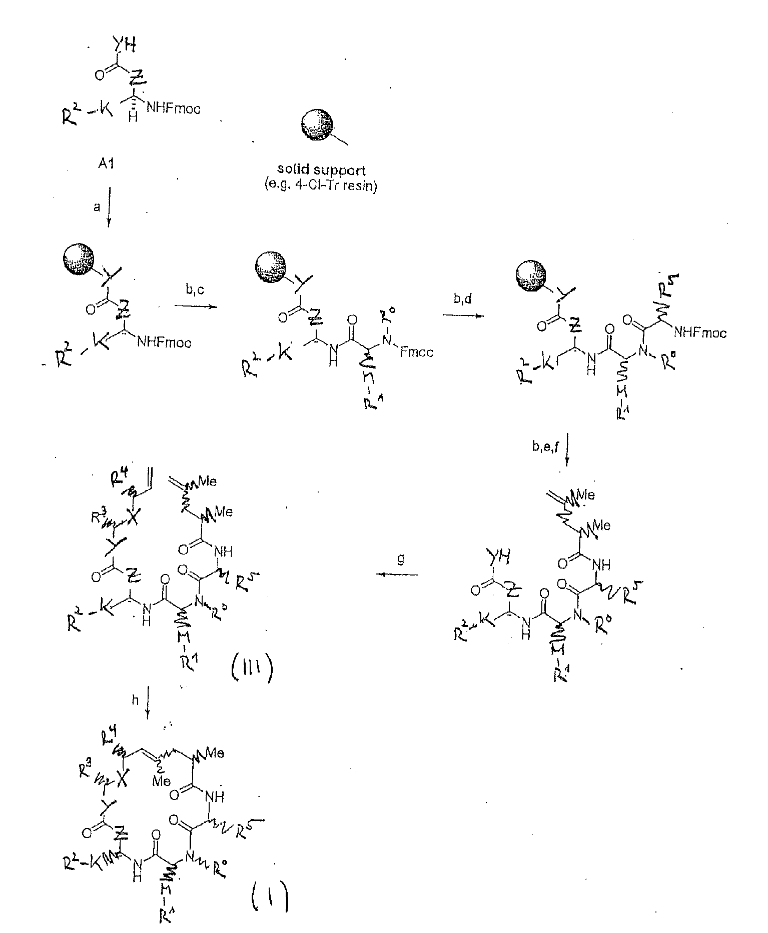 **Scheme 1: Synthesis of compound (III);** *Reagents and conditions:* (a) **(A1),** 2-chlorotrityl chloride polystyrene resin, (*i*Pr)₂NEt, CH₂Cl₂, rt, 1 h (loading 0.5-1 mmol/g); (b) DBU, piperidine, NMP (5 x), rt; (c) Fmoc-AA-OH, DIC, HOBt, DMF, rt, 2-3 h; (d) Fmoc-AA-OH, HATU, HOAt, (*i*Pr)₂NEt, NMP, rt, 2-3 h; (e) 2,4-dimethyl-4-pentenoic acid, DIC or HATU, HOBt, (*i*Pr)₂NEt, NMP, rt, 2-3 h; (f) HFIP/CH₂Cl₂ (1 x 20 min, 1 x 10 min); (g) R-YH, EDC·HCl, (*i*Pr)₂NEt, DMAP, CH₂Cl₂/DMF, rt, 14 h; (h) 5-20% metathesis catalyst, CH₂Cl₂, 40°C, Ar purging, 2-6h.

After the metathesis reaction the obtained products are optionally deprotected and further reacted with an optionally activated diagnostic and/or therapeutic agent, thereby forming an amide, ester, ether, thioether, amine, carbonate, carbamate urea or semicarbazide linkage, preferably an amide linkage.

It was surprisingly found that the toxicity of the compounds according to the present invention could be reduced compared to the naturally occurring compound 1 (Figure 1).

Moreover, it has been found that the synthetic cyclodepsipeptides according to the present invention provide powerful tools for targeting and imaging actin, particularly actin filaments. Characteristic and intense labelling of actin-rich structures has been achieved in a highly practical fashion. It was surprisingly found that labelling of actin-rich structures is not only possible in histological sections or bulk tissue but also in live cells.

The low toxicity, high propensity to enter into living cells and reduced interference with cytoskeletal dynamics makes ideal probes for imaging actin structures and dynamic processes in live cells of the compounds according to the invention. It has been found that particularly the substituent R⁵ exhibiting a spacer to which the diagnostic agent is bound seems to modify the compounds such that binding to actin and cell permeability are improved, while cytotoxicity can be reduced or at least maintained.

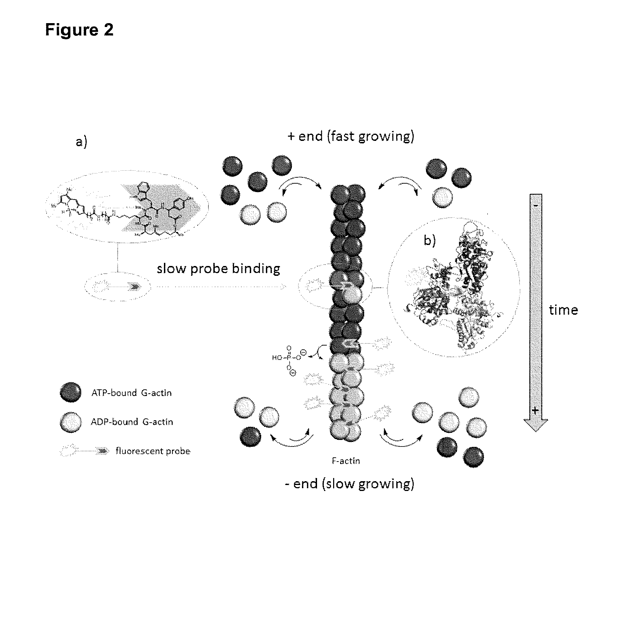

It was found that particularly the long-lived filament actin can be visualized with high sensitivity upon standard and readily performed incubation and washing procedures without recourse to genetic or single-cell manipulations. During actin polymerization, globular monomeric actin (G-actin) loaded with ATP preferentially adds at the fast-growing plus-ends of existing F-actin filaments (F-actin) (Fig. 2A). Without sticking to any theory, the dye-labelled probe is expected to bind F-actin with a relatively slow on-rate due to the sterically encumbered binding environment at the tip of three actin protein monomers (see Fig. 2B). As a result the dye-labelled probes stain highly dynamic short-lived filament populations such as in lamellipodia or filopodia considerably weaker compared to long lived filament populations such as those in stress fibres (Schaefer et al. J. Cell. Biol. 2002, 158, 139-152).

Thus, in another aspect of the invention the compounds according to the invention are used for imaging and/or targeting actin, particularly actin filaments.

The compounds according to the invention may be used for imaging actin in histological sections or bulk tissues. In particular because of their high cell permeability and their low cytotoxicity they are also suitable for cell imaging of actin in live cells. As shown above, the compounds according to the invention are particularly suitable for imaging static actin filaments and may be used for selectively imaging static actin filaments against dynamic actin populations, e.g. dynamic F-actin and monomeric G-actin populations.

In another embodiment, the compounds according to the invention that exhibit a therapeutic agent may be used to target actin structures. The therapeutic agent may be selectively transported to the locus in need thereof and may then either act from that location or be enzymatically, hydrolytically or pH-dependently cleaved off, e.g. at the coupling group CG.

The compounds and probes according to the invention can be generally accessed using an efficient and scalable total synthesis protocol, which potentially supersedes the complicated phalloidin derivatives based on material from difficult-to-cultivate sources.

The invention is described in more detail in the following Examples.

### Figure Legend

**Figure 1****.** Actin stabilizing compounds and fluorescently-labelled phalloidin conjugates **1-5,** simplified cyclodepsipeptide analogs **6-8** and Boc-protected lysine analogs **9-13** for dye conjugation studies. Fluorescently-labelled conjugates **19-22/A-D.**
**Figure 2****.** a) Simplified schematic overview of the actin polymerization process and the preferred staining of long-lived actin structures. b) Proposed binding model (Waldmann et al. Angew. Chem lnt. Ed. 2008, 47, 6473-6477). The cyclodepsipeptide ligand binds to and stabilizes the actin filaments at a point of contact between three G-actin monomers, with the dye presumably protruding into the solvent. Due to the slow on-rate of probe binding to F-actin, short-lived, more dynamic actin structures are labeled much weaker compared to long-lived, less dynamic actin structures.

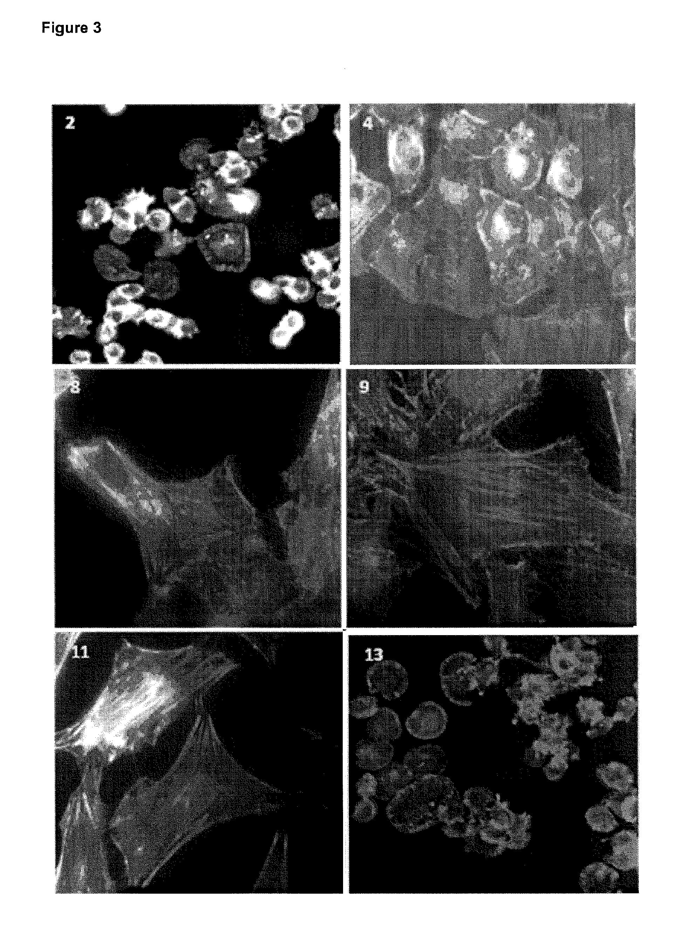

**Figure 3****.** Representative immunostaining data. **2** fixed, non-permeabilized non-transfected Neuro-2a cells treated @ 10 µM respectively with **20C** and **21C; 4** fixed, non-permeabilized BSC-1 cells treated, **21C** @ 30 nM; **8** and **9** fixed, non-permeabilized HeLa cells respectively treated with **19C** and **21C** @ 1 µM; **11** live HeLa cells treated with **19D** @ 1 µM after 11 h of incubation. **13** Fixed, non-permeabilized Neuro-2a cells stained with **19B.** Alternative to our standard labeling protocol (amino group deprotection → dye coupling → purification by preparative HPLC), N2a cells were treated with **19B** immediately after dye coupling, after quenching the unreacted fluorescein dye label with an excess of ethanolamine.

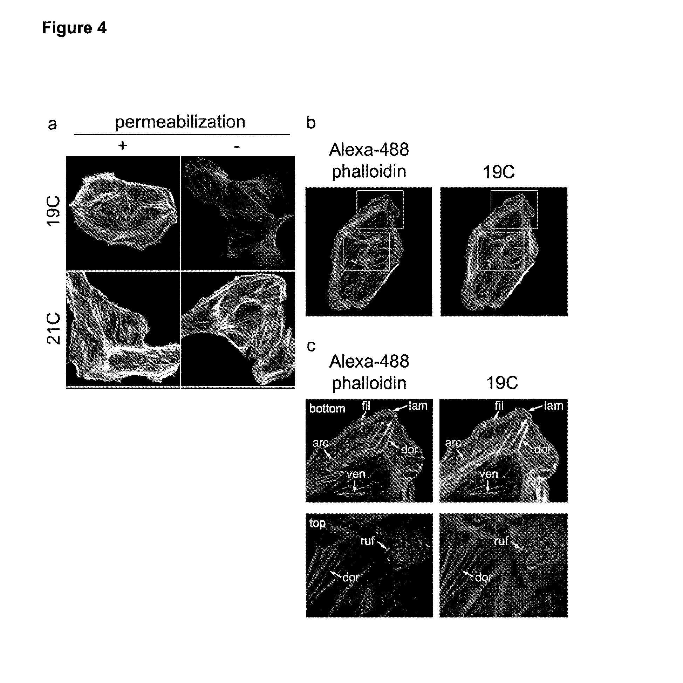

**Figure 4****.** Representative images of fixed U2OS cells stained with labeled probes **19C** and **21C.** A) U2OS cells were fixed and stained either with (+) or without (-) additional cell permeabilization and imaged using confocal microcopy. B) Two-channel analysis of fixed and permeabilized U2OS cells costained with Alexa-488 phalloidin and **19C** (1 µM) in maximum projections of z-stacks). C) Direct comparison of individual z-sections near (bottom) and far (top) from the glass substrate in two regions of interest highlighted in B. Due to their different spectral properties, the resolution in the z-plane was lower for the red-shifted TAMRA dye than for Alexa-488. Therefore, more structures were visible outside the plane of focus when using the TAMRA probe **19C.** Abbreviations: filopodia (fil), lamelipodia (lam), transversal arcs (arc), dorsal stress fibres (dor), ventral stress fibres (ven) and dorsal ruffles (ruf).

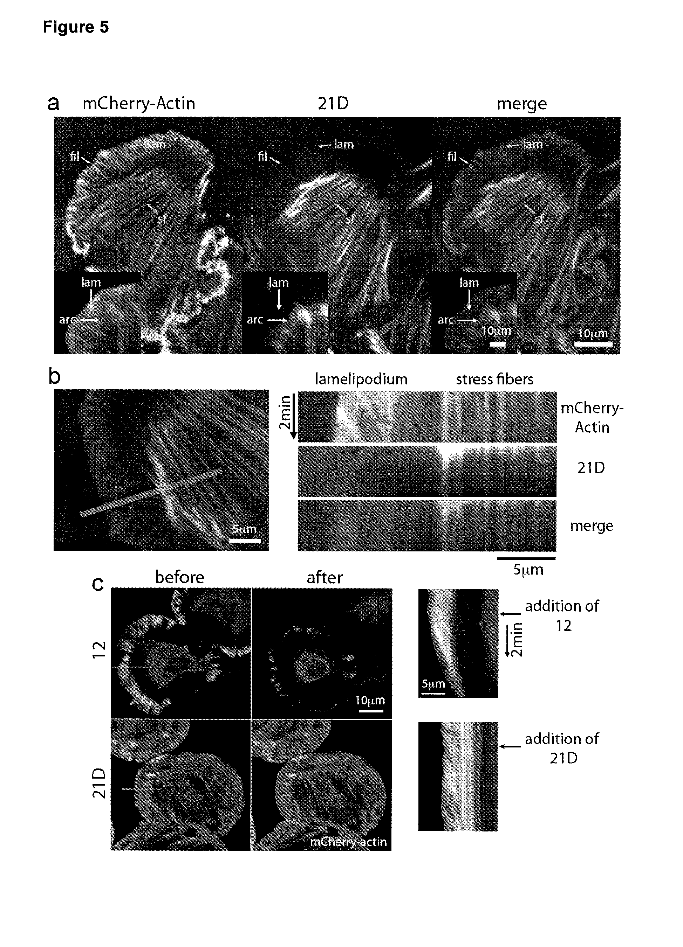

**Figure 5****.** Representative images of living Neuro-2a cells treated with probes **12** and **21 D.** (a) Living Neuro-2a cells co-expressing a non-labeled dominant positive mutant (Q61 L) of Rac1 and actin fused to mCherry were incubated with **21 D** (10 µM) for 5 minutes in imaging medium, extensively washed, and subjected to TIRF microscopy. (b) Kymograph analysis of dynamic and static actin structures. Left: Line indicating the cell region used for kymograph analysis. Right: Kymograph of mCherry-actin shows dynamic retrograde actin flow in lamellipodia (diagonal lines) and stable actin structures in stress fibers (vertical lines). Only the stable actin structures are labeled with **21 D.** (c) Effect of non-labeled **12** (unlabelled) and **21 D** (BODIPY labeled) on actin dynamics. Left: Images (mCherry actin channel) before and 5 mins after compound application. Right: Kymograph analysis of actin dynamics during compound application.

### Abbreviations

- TIPS: triisopropylsilyl
- Boc: tert-butyloxycarbonyl
- Fmoc: fluorenylmethyloxycarbonyl
- Cbz: carbobenzyloxy
- Ts: tosyl
- Alloc: allyloxycarbonyl
- TAMRA: carboxytetramethylrhodamine
- BODIPY: boron-dipyrromethene
- BODIPY-FL-X: 6-(4,4-difluoro-5,7-dimethyl-4-bora-3a,4a-diaza-sindacene-3-propionyl)amino)hexanoic acid
- DBU: diazabicycloundecen
- HOBt: 1-hydroxybenzotriazole
- DMF: dimethylformamide
- HATU: 2-(1 H-7-Azabenzotriazol-1-yl)-1,1,3,3 tetramethyluronium hexafluorophosphate
- HOAt: 1-hydroxy-7-azabenzotriazole
- NMP: N-methylpyrrolidone
- DIC: N, N'-diisopropylcarbodiimide
- HFIP: hexafluoroisopropanol
- EDC Hcl: 1-Ethyl-3-(3-dimethylaminopropyl)carbodiimidehydrochloride
- DMAP: dimethylaminopyridine
- TBAF: tetra-N-butylammonium fluoride

### EXAMPLES

### Chemical Synthesis

### General Methods

All solvents, when not purchased in suitable purity or dryness, were distilled using standard methods (Small small et al. Microsc. Res. Tech. 1999, 47, 3-17). Alternatively, solvents (HPLC grade) were passed under dry argon atmosphere through activated alumina columns (M.Braun solvent purification system). Deionized water was used for all experiments. All reagents were purchased from commercial suppliers (Acros, Alfa Aesar, Fluka, Novabiochem, Sigma-Aldrich) and used without purification. 5-carboxy-fluorescein diacetate N-succinimidyl ester was purchased from Sigma-Aldrich. 5-(and 6-) carboxytetramethylrhodamine (TAMRA) N-succinimidyl ester purchased from EMP Biotech GmbH. BODIPY FL-X N-succinimidyl ester was purchased from Invitrogen.

Within a description of a reaction workup the term "concentration" refers to removal of the solvent under reduced pressure using a rotary evaporator, followed by removing residual volatiles at high vacuum (*p* < 0.1 mbar). Analytical Thin Layer Chromatography (TLC) was carried out on Merck precoated silica gel plates (60F-254) using ultraviolet light irradiation at 254 nm or KMnO4 solution as staining reagent (1 g KMnO₄, 6.6 g K₂CO₃, 1.7 mL 5% NaOH solution, 100 mL H₂O).

Column chromatography was performed using silica gel from J. T. Baker (particle size 40 µm - pore size 60 A), either gravimetrically or under a pressure of 0.3-0.5 bar. Column dimensions are described by height (h) and diameter (d). Capillary Gas Chromatography (GC) was conducted using a HP 6890 GC system equipped with a HP-5MS column (24.8 m x 201 µm x 0.33 µm). Analytical HPLC (aHPLC in the text) was performed on an Agilent 1100 machine using a Macherey-Nagel C18 gravity 3 µm Reversed Phase column. The separations were started at 10% MeCN (with 0.1% TFA) in H₂O (with 0.1% TFA) with a flow of 1 mL·min⁻¹, and the MeCN proportion was linearly increased after 1 min to 100% over a period of 7 min and then kept at that proportion for a period of 7 min. Preparative HPLC was performed on a Waters machine using a Macherey-Nagel C18 gravity 5 µm reversed phase column. The separations were started at 10% MeCN (with 0.1 % TFA) in H₂O (with 0.1% TFA), and the MeCN proportion was linearly increased to 100 % over a period of 20 min with a flow of 20 mL·min⁻¹ (Method A). Alternatively, an Agilent system equipped with a Macherey Nagel C18 gravity 5 µm Reversed Phase column was used to perform isochratic separations at 50% MeCN in H₂O (with 0.1% TFA) applying a flow of 20 mL·min⁻¹ (Method B).

Melting points were determined with a Büchi Melting Point B-540 apparatus (uncorrected). Optical rotations were measured in a Schmidt + Haensch Polartronic HH8 polarimeter at 589 nm, with values given in 10⁻¹ deg cm² g⁻¹ and concentrations c given in g/100mL. ¹H- and ¹³C-NMR spectra were recorded on Varian Unity Inova 600 (599.8 MHz (¹H) and 150.8 MHz (¹³C)), Bruker DRX 500 (500.1 MHz (¹H) and 125.8 MHz (¹³C)), Bruker DRX 400 (400 MHz (¹H), 100.5 MHz (¹³C) and 188 MHz (¹⁹F)) and Varian Mercury VX 400 (400.1 MHz (¹H) and 100.6 MHz (¹³C)) spectrometers. Chemical shifts are expressed in parts per million (ppm) and the spectra are calibrated to residual solvent signals of CDCl₃ (7.26 ppm (¹H) and 77.0 ppm (¹³C)), CD3OD (3.31 ppm (1H) and 49.0 ppm (13C)), C₆D₆ (7.16 ppm (¹H) and 128.0 ppm (¹³C)), DMSO (2.50 ppm (¹H) and 39.43 ppm (¹³C)), D₂O (4.79 ppm (¹H)) and CF₃COOH (-77.46 ppm (¹⁹F)) respectively. Coupling constants are given in Hertz (Hz) and the following notations indicate the multiplicity of the signals: s (singlet), d (doublet), t (triplet), q (quartet), qui (quintet), sext (sextet), sept (septet), m (multiplet), app (apparent), br (broad signal). Unless otherwise stated, spectra were recorded at 27°C. Fourier transform infrared spectroscopy (FT-IR) spectra were obtained with a Bruker Tensor 27 spectrometer (ATR, neat or as a thin film). Low Resolution Mass Spectra were recorded on a Thermo Finnigan LCQ ESI spectrometer (source voltage 70 keV). High Resolution FAB Spectra were recorded on a Jeol SX 102 A (matrix: meta-nitrobenzyl alcohol). A Thermo DFS High Resolution Magnetic Sector MS (Double Focusing Mass Spectrometer) device was used to record EI (source voltage 70 keV; reference substance: perfluorokerosene, resolution: 10000) and Cl (methane gas; source voltage 120 keV; reference substance: Ultramark 2500F, resolution: 10000) spectra, respectively. High Resolution ESI Spectra were recorded on a Thermo Electron LTQ Orbitrap (source voltage 3.8 kV, resolution: 60000) spectrometer.

### Synthesis of Fluorescent Actin Probes

In scheme 2 a) the synthesis of fluorescent actin probes is schematically shown based on compounds 9-13 (Figure 1). It is clear to the skilled worker that the coupling of diagnostic agents can be performed in an analogous manner.

### Synthesis of fluorescent actin probes

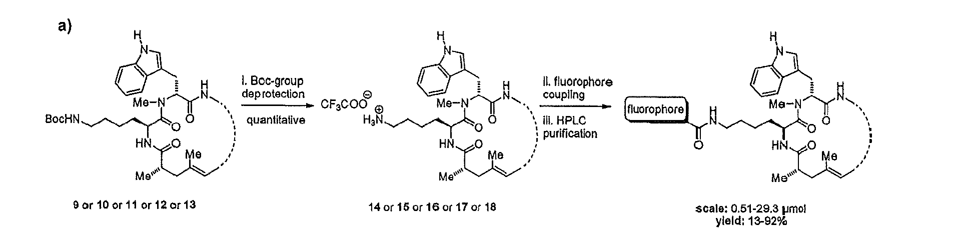

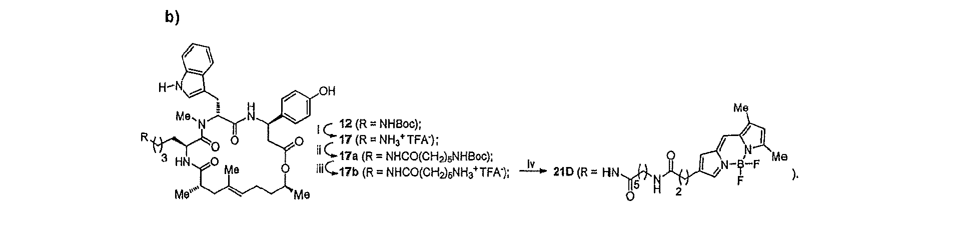

**Scheme 2. a). Synthesis of dye conjugates:** *Reagents and conditions:* i. 30% CF₃COOH/CH₂Cl₂ (v/v), 1 h, r.t.; ii. fluorophore NHS ester, (*i*Pr)₂NEt, DMSO, r.t., 4 h; iii. semiprep. HPLC purification 13-92 %. b). Synthesis of 21 D: *Reagents and conditions:* i. 30% CF₃COOH/CH₂Cl₂ (v/v), 1 h, r.t.; ii. 6-(*tert*-butoxycarbonylamino) hexanoic acid, HOAt, HATU, N-methylmorpholine, DMSO, r.t., 14 h; iii. 30% CF₃COOH/CH₂Cl₂ (v/v), 1 h, r.t.; iv. BODIPY® FL propionic acid (4,4-difluoro-5,7-dimethyl-4-bora-3a,4a-diaza-s-indacene-3-propionic acid), HOAt, HATU, *N*-methylmorpholine, DMSO, 14 h, r.t., 26 % over 4 steps.

### Synthesis of Lysine Analogues

Figure 1 shows several compounds according to the present invention, which are obtained according to the methods as described below.

### cyclo-[Lys(Boc)-D-MeTrp-βTyr(OTIPS)-(2S,4E,6R,7R)-Hto] (9)

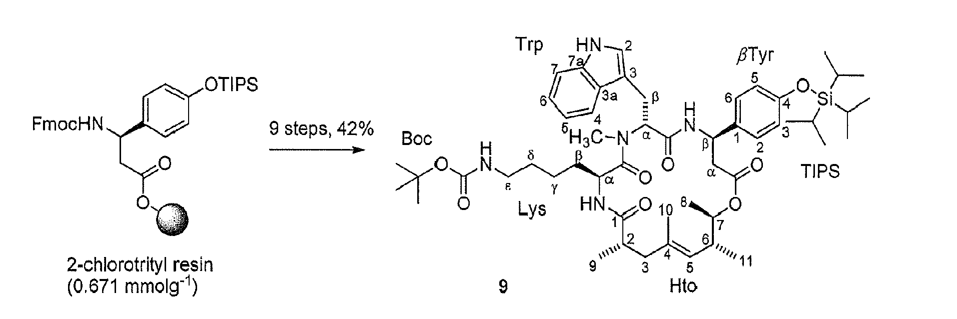

Cyclodepsipeptide **9** was obtained according to the procedure described for the synthesis of compound **13c**/(O-TIPS)-chondramide C by Tannert et al. (J. Am. Chem. Soc., 2010, 132, 3063) by replacing Fmoc-Lys-(Boc)-OH with Fmoc-Ala-OH for the synthesis of the tripeptide motif. Column chromatography of the crude (EtOAc/cyclohexane 1:1), followed by semi-preparative RP-HPLC (method A), yielded **9** (6.2 mg, 42 % overall yield based on the loading of the first amino acid (0.671 mmol·g⁻¹)) as a white powder after freeze-drying. R_{f} = 0.58 (EtOAc/cyclohexane 3:2); **aHPLC** (see general methods): (LC: tR = 12.55 min); **LCMS** (C18): (LC: tR = 12.29 min); [α]_{D}²⁰ = +65.6 (c = 0.11 in CHCl3); **¹H NMR** (400 MHz, CDCl3): δ = 9.89 (s, 1H; Trp2-NH), 7.58 (d, ³J = 8.0 Hz, 1H; Trp4-H), 7.33 (d, ³J = 8.0 Hz, 1H; Trp7-H), 7.19-7.13 (m, 3H; βTyr2-H, βTyr6-H, Trp6-H), 7.11-7.03 (m, 2H; Trp5-H, βTyrrβ-NH), 6.88 (s, 1H; Trp2-H), 6.84 (d, ³J = 8.4 Hz, 2H; βTyr3-H, βTyr5-H), 6.50 (d, ³J = 6.8 Hz, 1H; Lys-NH), 5.55 (t, ³J = 10.8 Hz, 1H; Trpα-H), 5.43 (dd, ³J = 12.8/4.4 Hz, 1H; βTyrrβ-H), 4.95 (d, ³J = 9.2 Hz, 1H; Hto5-H), 4.75-4.66 (m, 3H; Hto7-H, Lysα-H, Boc-NHC(O)OC(CH₃)₃), 3.43 (dd, ³J = 12.8 Hz, ²J=16.8 Hz, 1H; Trpβ-Ha), 3.16 (dd, ³J = 4.0 Hz, ²J=16.8 Hz, 1H; Trpβ-H_{b}), 3.04-2.99 (m, 1H; Lysε-Hₐ), 2.88-2.84 (m, 1H; Lysε-H_{b}), 2.83 (s, 3H; Trp-NCH₃), 2.69 (dd, ³J = 2.4 Hz, ²J = 15.6 Hz, 1H; βTyrα-Hₐ), 2.58-2.42 (m, 4H; Hto2-H, βTyrα-H_{b}, Hto6-H, Hto3-Hₐ), 1.87 (app. d, ²J = 11.2 Hz, 1H; Hto3-H_{b}), 1.63 (s, 3H; Hto10-H3), 1.53 (s, 9H; Boc-NHC(O)OC(CH₃)₃), 1.29-1.20 (m, 6H; TIPS, Lysβ-Hₐ, Lysδ-H), 1.16 (d, ³J = 6.0 Hz, 3H; Hto8-H3), 1.12-1.06 (m, 1H; Lysrβ-H_{b}), 1.09 (d, ³J = 7.2 Hz, 18H; TIPS), 1.01 (d, ³J = 6.4 Hz, 3H; Hto11-H3), 0.89 (d, ³J = 6.8 Hz, 3H; Hto9-H3), 0.90-0.76 (m, 2H; Lysy-H) ppm; **¹³C NMR** (100 MHz, CDCl₃): δ = 174.5, 174.2, 170.5, 169.2, 156.8, 155.6, 136.6, 134.03, 133.96, 128.8, 127.3, 127.2 (2C), 122.0, 121.5, 120.1 (2C), 119.2, 118.5, 111.6, 110.1, 80.0, 77.5-76.8 (signal obscured by solvent peak), 56.7, 49.6, 49.2, 45.0, 42.6, 40.9, 40.7, 36.3, 32.0, 30.5, 30.1, 29.8, 28.6 (3C), 22.4, 21.4, 19.8, 18.1 (6C), 16.7, 15.1, 12.8 (3C) ppm; **IR** (neat): v^{∼} = 3305, 2933, 2867, 1682, 1638 cm⁻¹; HR-MS (ESI): calc. for C₅₂H₈₀N₅O₈Si [M+H]⁺: 930,5776, found 930,5775; calc. for C₅₂H₇₉N₅NaO₈Si [M+Na]⁺: 952,5596, found 952,5583.

### cyclo-[Lys(Boc)-D-MeTrp-βTyr-(2S,4E,6R,7R)-Hto] (10)

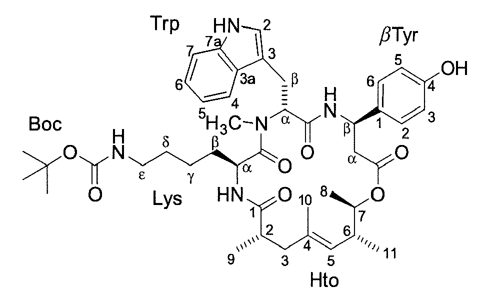

The desilylation of **9** (4.9 mg, 5.3 µmol) was performed in analogy to the procedure described for compound **14c** by Tannert et al. Column chromatography (EtOAc/cyclohexane 3:2 to 4:1) of the crude product followed by semi-preparative RP-HPLC (method A) yielded 10 (4.1 mg, quantitative) as a white powder after freeze-drying. ***R***_{f} = 0.30 (EtOAc/cyclohexane 4:1); **aHPLC:** (LC: *t*_{R} = 7.96 min); **LCMS** (C18): (LC: *t*_{R} = 10.13 min); **[α]_{D}**²⁰=+58.5 (c = 0.07 in CHCl₃); ¹H **NMR** (400 MHz, CDCl₃): δ = 9.82 (br s, 1H; Trp2-NH), 7.57 (d, ³*J* = 7.6 Hz, 1H; Trp4-H), 7.32 (d, ³*J* = 8.0 Hz, 1H; Trp7-H), 7.16-7.06 (m, 5H; *β*Tyr3-*H*, *β*Tyr5-*H*, Trp5-*H*, Trp7-H, *β*Tyr*β*-N*H*), 6.88 (d, ³*J* = 2.0 Hz, 1H; Trp2-H), 6.79 (d, ³*J* = 8.8 Hz, 2H; *β*Tyr3-H, *β*Tyr5-*H*), 6.48 (d, ³*J* = 8.8 Hz, 1H; Lysα-N*H*), 5.51-5.45 (m, 2H; *β*Tyrβ-*H*, Trpα-*H*), 4.94 (d, ³*J* = 9,2 Hz, 2H; Hto5-*H*), 4.77 (br t, ³*J* = 4.0 Hz, 1H; Boc-N*H*C(O)OC(CH₃)₃), 4.74-4.65 (m, 2H; Lysα-*H*, Hto7-*H*), 3.39 (dd, ³*J* = 10.8 Hz, ²*J* = 15.6-18.0 Hz, 1H; Trp*β*-*H*ₐ), 3.19 (dd, ³*J* = 4.8 Hz, ²*J* = 16.8 Hz, 1H; Trpβ-*H*_{b}), 3.01-2.94 (m, 1H; Lysε-*H*ₐ), 2.89-2.82 (m, 1H; Lysε-*H*_{b}), 2.85 (s, 3H; Trp-NC*H*₃), 2.65 (dd, ³*J* = 2.8 Hz, ²*J* = 15.2 Hz, 1H; *β*Tyrα-*H*ₐ), 2.58-2.43 (m, 4H; Hto2-*H*, *β*Tyrα-*H*_{b}, Hto6-*H*, Hto3-*H*ₐ), 1.90 (d, ³*J* = 10.8 Hz, 1H; Hto3-*H*_{b}), 1.63 (s, 3H; Hto10-*H*₃), 1.52 (s, 9H; Boc-NHC(O)OC(C*H*₃)₃), 1.47-1.21 (m, 3H; Lysβ-*H*ₐ, Lysδ-*H*), 1.16 (d, ³*J* = 6.0 Hz, 3H; Hto8-*H*₃), 1.12-1.06 (m, 1H; Lysβ-*H*_{b}), 1.01 (d, ³*J* = 6.4 Hz, 3H; Hto11-*H*₃), 0.89 (d, ³*J* = 6.8 Hz, 3H; Hto9-*H*₃), 0.93-0.77 (m, 2H; Lysy-*H*) ppm; ¹³**C NMR** (100 MHz, CDCl₃): δ = 174.9, 174.4, 170.7, 169.4, 156.8, 155.9, 136.6, 134.0, 133.4, 128.6, 127.5 (2C), 127.3, 122.0, 121.7, 119.3, 118.5, 115.8 (2C), 111.5, 109.9, 80.0, 77.5-76.8 (signal obscured by solvent peak), 56.9, 49.5, 45.1, 42.5, 40.8, 40.6, 36.3, 32.1, 30.5, 30.0, 29.9, 28.5 (3C), 22.6, 21.4, 19.7, 17.9, 16.7, 15.2 ppm.; **IR** (neat): *v*^{∼} = 3308, 3056, 2960, 2924, 2854, 1717, 1663, 1636 cm⁻¹; **HR-MS** (ESI): calc. for C₄₃H₆₀N₅O₈ [M+H]⁺: 774.4442, found 774.4440; calc. for C₄₃H₅₉N₅NaO₈ [M+Na]⁺: 796.4261, found 796.4247.

### cyclo-[Lys(Boc)-D-MeTrp-βTyr(OTIPS)-(2S,4E,8S)-Htn] (11)

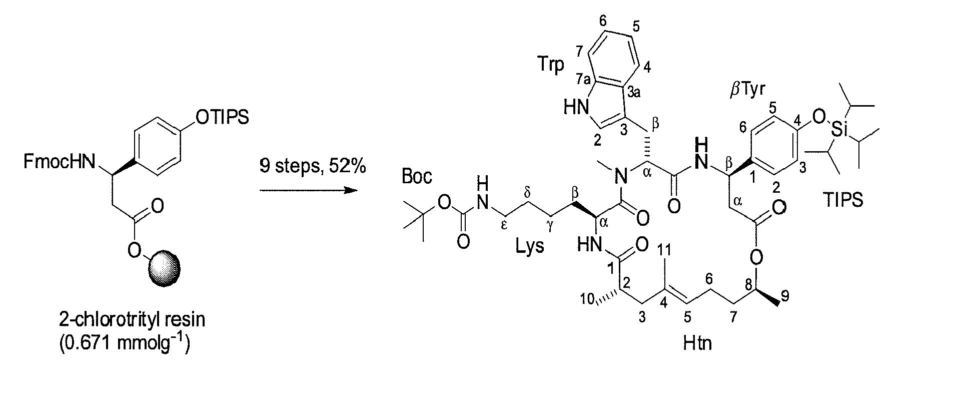

Cyclodepsipeptide **11** was obtained according to the procedure described for the synthesis of compound ***E*-30c** by Tannert et al. by replacing Fmoc-Lys-(Boc)-OH with Fmoc-Ala-OH for the synthesis of the tripeptide motif. Column chromatography (EtOAc/cyclohexane 1:1 to 3:2) of the crude followed by semi-preparative RP-HPLC (method A) yielded **11** (26.8 mg, 52 % overall yield based on the loading of the first amino acid (0.671 mmol·g⁻¹) as a white powder after freeze-drying. ***R*_{f}** = 0.25 (EtOAc/cyclohexane 3:2); **aHPLC:** (LC: *t*_{R} = 13.60 min + minor peak *t*_{R} = 13.19, ca. 10% - LCMS spectrum shows only one peak and one (correct) mass. Therefore, minor peak presumed to be the Z-isomer); **LCMS** (C18): (LC: *t*_{R} = 12.08 min, one peak); **[α]_{D}²⁰** = +61.5 (c = 0.07 in MeOH); **¹H NMR** (400 MHz, CDCl₃): δ = 9.88 (br s, 1H; Trp2-NH), 7.61 (d, ³*J* = 7.6 Hz, 1H; Trp4-H), 7.37-7.33 (m, 2H; Trp7-H, *β*Tyr-NH), 7.17-7.08 (m, 4H, *β*Tyr3-*H*, *β*Tyr5-*H*, Trp5-*H*, Trp7-H), 6.95 (d, ³*J* = 1.6 Hz, 1H; Trp2-H), 6.82 (d, ³*J* = 8.4 Hz, 2H; Tyr, Tyr), 6.58 (d, ³*J* = 6.8 Hz, 1H; Lys-NH), 5.69 (dd, ³*J* = 12.4, 4.8 Hz, 1H; Trpα-*H*), 5.26-5.21 (m, 1H; *β*Tyrβ-H), 5.00 (t, ³*J* = 7.2 Hz, 1H; Htn5-*H*), 4.86-4.83 (m, 1H), 4.83-4.78 (m, 1H), 4.73 (t, ³*J* = 5.6 Hz, 1H; Boc-N*H*C(O)OC(CH₃)₃), 3.38 (dd, ³*J* = 12.4 Hz, ²*J* = 16.4 Hz, 1H; Trpβ-*H*ₐ), 3.28 (dd, ³*J* = 4.4 Hz, ²*J* = 16.4 Hz, 1H; Trpβ-*H*_{b}), 3.10-2.99 (m, 1H; Lysε-*H*ₐ), 2.89 (s, 3H; Trp-NC*H*₃), 2.87-2.76 (m, 1H; Lysε-*H_{b}),* 2.78 (dd, ³*J* = 4.0 Hz, ²*J* = 15.6 Hz, 1H; *β*Tyrα-*H*ₐ), 2.57 (dd, ³*J* = 7.6 Hz, ²*J* = 15.6 Hz, 1H; *β*Tyrα-*H*_{b}), 2.49-2.42 (m, 2H), 1.86-1.78 (m, 3H), 1.69-1.65 (m, 2H), 1.64-1.55 (m, 2H), 1.55-1.49 (m, 2H), 1.53 (s, 9H), 1.49-1.44 (m, 1H), 1.46 (s, 3H), 1.37-1.18 (m), 1.17-1.05 (m), 1.05-0.98 (m, 2H), 0.90-0.82 (m, 1H), 0.73-0.60 (m, 1H) ppm; **¹³C NMR** (100 MHz, CDCl₃): δ = 174.6, 174.1, 170.6, 169.5, 156.8, 155.6, 136.7, 134.1, 133.1, 127.3 (2C), 124.6, 121.9, 121.7, 120.1 (2C), 119.2, 118.5, 111.6, 110.4, 79.9, 77.5-76.8 (signal obscured by solvent peak), 69.6, 56.2, 49.9, 49.2, 43.4, 40.9, 39.9, 39.8, 35.7, 31.0, 30.4, 30.3, 29.9, 28.6 (3C), 23.3, 22.5, 21.2, 20.6, 20.5, 18.1 (6C), 16.4, 12.8 (3C) ppm. **IR** (neat): *v*^{∼} = 3310, 3057, 2929, 2867, 1684, 1637 cm⁻¹; **HR-MS** (ESI): calc. for C₅₂H₈₀N₅O₈Si [M+H]⁺: 930.5776, found 930.5778; calc. for C₅₂H₇₉N₅NaO₈Si [M+Na]⁺: 952.5596, found 952.5586.

### cyclo-[Lys(Boc)-D-MeTrp-βTyr-(2S,4E,8S)-Htn] (12)

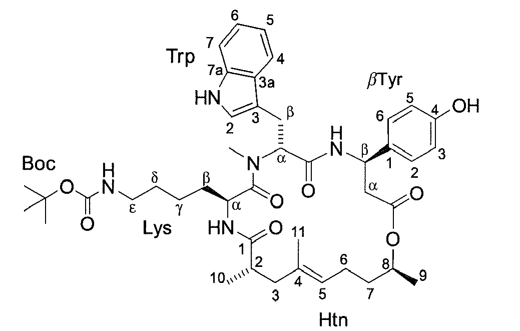

The desilylation of **11** (42 mg, 45.1 µmol) was performed according to the procedure described for compound **10.** Column chromatography (EtOAc/cyclohexane 3:2 →4:1) of the crude followed by semi-preparative RP-HPLC (method A) yielded **12** (33.3 mg, 79%) as a white powder after freeze-drying. ***R*_{f}** = 0.19 (EtOAc/cyclohexane 7:3); **aHPLC:** (LC: *t*_{R} = 7.97 min); **LCMS** (C18): (LC: *t*_{R} = 10.55 min.); [*α*]_{D}²⁰ = +37.5 (c = 0.07 in MeOH); **¹H NMR** (600 MHz, [D₇]DMF): E/Z-mixture (> 9:1 ratio), only E-isomer evident: δ = 10.81 (br s, 1H; Trp2-NH), 9.48 (br s, 1H; Tyr-OH), 8.47 (d, ³*J* = 8.4 Hz, 1H; βTyr-N*H*), 7.66 (d, ³*J* = 7.8 Hz, 1H; Trp-H), 7.39 (apparent d, ³*J* = 7.8 Hz, 2H; Trp-H and Lys-NH), 7.20 (d, ³*J* = 8.4 Hz, 2H; Tyr-*H₂*), 7.15 (s, 1H; Trp2-H), 7.08 (t, ³*J* = 7.2 Hz, 1H; Trp-H), 6.98 (t, ³*J* = 7.8 Hz, 1H; Trp-H), 6.79 (d, ³*J* = 9.0 Hz, 2H; Tyr-*H*₂), 6.62 (br t, ³*J* = 4.8 Hz, 1H; Boc-N*H*C(O)OC(CH₃)₃), 5.64 (dd, ³*J* = 10.2, 6.0 Hz, 1H; Trpα-*H*), 5.34-5.31 (m, 1H; βTyra-*H*), 5.05 (t, ³*J* = 6.6 Hz, 1H; Htn5-*H*), 4.79-4.74 (m, 2H; Htn8-*H* and Lysα-*H*), 3.19 (dd, ³*J* = 6.0 Hz, ²*J* = 15.0 Hz, 1H; Trpβ-*H*ₐ), 3.16-3.11 (m, 1H; Trpβ-*H*_{b}), 3.15 (s, 3H; Trp-NC*H*₃), 2.90-2.83 (m, 4H; Lysε-*H*₂, βTyrβ-*H*, Htn2-H), 2.71 (dd, ³*J* = 3.6, ²*J* = 15.6 Hz, 1H), 2.68-2.63 (m, 1H), 2.27 (apparent t, ³*J* = 13.8 Hz, 1H), 1.91 (q, ³*J* = 7.2 Hz, 2H), 1.85 (d, ³*J* = 14.4 Hz, 1H), 1.61-1.55 (m, 1H), 1.54 (s, 3H; Htn11-*H*₃), 1.46-1.38 (m, 1H), 1.41 (s, 9H; Boc-NHC(O)OC(C*H*₃)₃), 1.26-1.21 (m, 2H), 1.17 (d, ³*J* = 6.0 Hz, 3H), 1.16-1.10 (m, 1H), 1.05 (d, ³*J* = 6.6 Hz, 3H), 1.04-0.95 (m, 1H), 0.91-0.85 (m, 1H) ppm; **¹³C NMR** (150 MHz, [D₇]DMF) - calibration signal 162,62 for DMF-C=O: only E-isomer evident: δ = 175.3, 173.0, 171.0, 170.4, 157.4, 156.7, 137.3, 134.0, 133.9, 128.0, 127.9 (2C), 124.6, 124.1, 121.5, 119.1, 118.8, 115.7 (2C), 111.8, 110.7, 78.1, 71.1, 56.1, 49.9, 49.0, 43.9, 41.9, 40.6, 39.3, 35.9, 32.2, 30.9, 30.4-29.6 (signal obscured by solvent peak), 28.5 (3C), 25.7, 24.2, 22.5, 19.9, 19.7, 16.7 ppm; **IR** (neat): *v*^{∼} = 3404, 3307, 3077, 2927, 2865, 1714, 1686, 1655, 1635 cm⁻¹; **HR-MS** (ESI): calc. for C₄₃H₆₀N₅O₈ [M+H]⁺: 774,4442, found 774.4438; calc. for C₄₃H₅₉N₅NaO₈ [M+Na]⁺: 796,4261, found 796.4253.

### cyclo-[Lys(Boc)-D-MeTrp-βAla-(2S,4E,8S)-Htn] (13)

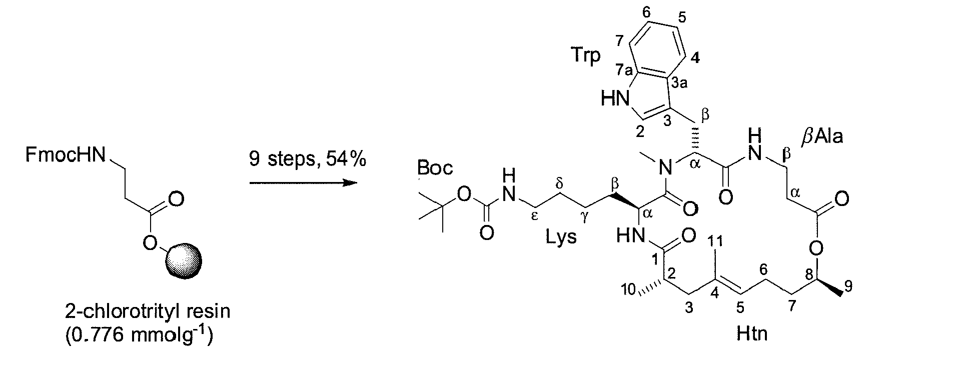

Cyclodepsipeptide 13 was obtained according to the procedure described for the synthesis of compound ***E*-31e** by Tannert et al. by replacing Fmoc-Lys-(Boc)-OH with Fmoc-Ala-OH for the synthesis of the tripeptide motif. Column chromatography (EtOAc/cyclohexane 4:1) of the crude followed by semi-preparative RP-HPLC (method A) yielded 13 (57.1 mg, 54 % overall yield based on the loading of the first amino acid (0.776 mmol·g⁻¹) as a white powder after freeze-drying. ***R*_{f}** = 0.19 (EtOAc/cyclohexane 4:1); **aHPLC**: (LC: *t*_{R} = 8.18 min); **LCMS**: (LC: *t*_{R} = 10.64 min); **[α]_{D}²⁰** = +114.0 (c = 0.07 in MeOH); **¹H NMR** (400 MHz, CDCl₃): δ = 9.87 (br s, 1H; Trp2-NH), 7.60 (d, ³*J* = 7.8 Hz, 1H; Trp), 7.34 (d, ³*J* = 8.4 Hz, 1H; Trp), 7.15 (t, ³*J* = 7.2 Hz, 1H; Trp), 7.09 (t, ³*J* = 7.2 Hz, 1H; Trp), 6.97 (d, ³*J* = 1.6 Hz, 1H; Trp2-H), 6.76 (t, ³*J* = 6.0 Hz, 1H; βAla-N*H*), 6.39 (d, ³*J* = 7,2 Hz, 1H; Lys-NH), 5.62 (dd, ³*J* = 11.4, 5.4 Hz, 1H; Trpα-*H*), 5.10 (t, ³*J* = 6,6 Hz, 1H; Htn*5-H*), 4.87-4.83 (m, 1H; Htn8-H), 4.75-4.72 (m, 2H; Lysα-*H* and Boc-N*H*C(O)OC(CH₃)₃), 3.56-3.48 (m, 2H; βAlaβ-*H₂*), 3.36 (dd, ³*J* = 4.8 Hz, ²*J* = 16.2 Hz, 1H; Trpβ-*H*ₐ), 3.32 (dd, ³*J* = 12.0 Hz, *²J* = 16.2 Hz, 1H; Trpβ-*H*_{b}), 3.04-2.99 (broad m, 1H; Lysε-Hₐ), 2.87 (s, 3H; Trp-NC*H*₃), 2.83-2.77 (broad m, 1H; Lysε-*H*_{b}), 2.58 (ddd, ³*J* = 4.2 and 16.8 Hz, ²*J* = 6.6 Hz, 1H; *β*Alaα-*H*ₐ), 2.44 (ddd, ³*J* = 7.8 and 16.8 Hz, ²*J* = 4.2 Hz, 1H; *β*Alaα-*H*ₐ), 2.38-2.30 (m, 2H, Htn2-*H* and Htn3-*H*ₐ), 1.99-1.94 (m, 1H), 1.91-1.85 (m, 2H), 1.72-1.66 (m, 1H), 1.52 (m, 12H; Htn11-*H*₃ and Boc-NHC(O)OC(C*H*₃)₃), 1.49-1.40 (m, 1H), 1.38-1.29 (m, 1H), 1.28-1.22 (m, 1H), 1.20 (d, ³*J* = 6.0 Hz, 3H; Htn9-*H*₃), 1.23-1.13 (m, 1H), 1.11 (d, ³*J* = 6.6 Hz, 3H; Htn10-*H*₃), 0.98-0.90 (m, 1H), 0.90-0.82 (m, 1H), 0.68-0.58 (m, 1H) ppm.; **¹³C NMR** (100 MHz, CDCl₃): δ = 175.1, 173.4, 171.6, 170.2, 156.7, 136.7, 134.2, 127.3, 124.7, 122.0, 121.9, 119.2, 118.5, 111.6, 110.2, 79.9, 77.5-76.8 (signal obscured by solvent peak), 70.9, 56.5, 49.5, 43.4, 40.7, 36.0, 35.3, 34.2, 31.3, 30.4, 30.2, 28.7 (3C), 23.9, 22.9, 21.2, 20.3, 19.7, 16.9 ppm.; **lR** (neat): 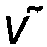 = 3309, 3056, 2973, 2931, 2867, 1712, 1638 cm⁻¹; **HR-MS** (ESI): calc. for C₃₇H₅₆N₅O₇ [M+H]⁺: 682.4180, found 682.4171; calc. for C₃₇H₅₅N₅NₐO₇ [M+Na]⁺: 704,3999, found 704.3985.

### Boc-group deprotection

### cyclo-[Lys-D-MeTrp-βTyr(TIPS)-(2S,4E,6R,7R)-Hto] trifluoroacetate salt (14)

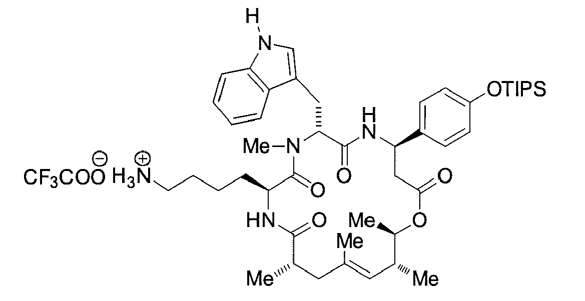

Boc-protected lysine analogue **9** (2.0 mg, 2.15 µmol) was pre-weighed in an oven-dried (100 °C, 24 h) 4 mL glass vial fitted with a magnetic stirrer, dissolved in 30% CF₃COOH/CH₂Cl₂ (0.25 mL, v/v) and stirred at r.t. for 1 h or when LC analysis showed complete conversion, which ever was sooner. The reaction was diluted with toluene (0.25 mL) and the solvents evaporated to constant dryness - first by purging with a continuous stream of dry argon, then overnight under high vacuum (p < 0.1 mbar) - to afford the crude trifluoroacetate salt **14** (off-white solid, 1.78 mg, quantitative), which was used directly for fluorophore coupling. **aHPLC:** (LC: *t*_{R} = 8.52 min); **LCMS** (C18): (LC: *t*_{R} = 10.17 min); **lR** (neat): 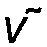 = 3293, 3056, 2961, 2926, 2866, 1670, 1636 cm⁻¹; **HR-MS** (ESI): calc. for C₄₇H₇₂N₅O₆Si [M+H]⁺: 830.5252, found 830.5237.

### cyclo-[Lys-D-MeTrp-βTyr-(2S,4E,6R,7R)-Hto] trifluoroacetate salt (15)

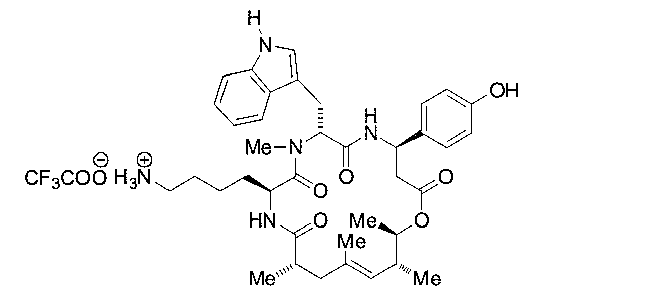

According to the procedure used for the synthesis of **14,** Boc-protected lysine analogue **10** (0.4 mg, 0.51 µmol) was converted to the crude trifluoroacetate salt **15** (pale colored wax, 0.34 mg, quantitative), and used directly for fluorophore coupling. **aHPLC:** (LC: *t*_{R} = 5.72 min); **LCMS** (C18): (LC: *t*_{R} = 7.77 min); **lR** (neat): 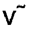 = 3357, 3193, 2960, 2924, 2853, 1660, 1633 cm⁻¹; **HR-MS** (ESI): calc. for C₃₈H₅₂N₅O₆ [M+H]⁺: 674.3918, found 674.3909; calc. for C₃₈H₅₁N₅NaO₆ [M+Na]⁺: 696.3737, found 696.3727.

### cyclo-[Lys-D-MeTrp-βTyr(OTIPS)-(2S,4E,8S)-Htn] trifluoroacetate salt (16)

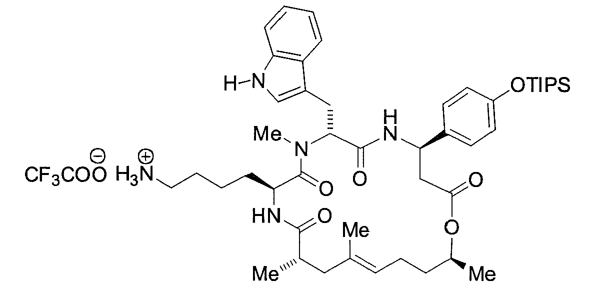

According to the procedure used for the synthesis of **14,** Boc-protected lysine analogue **11** (15.0 mg, 16.1 µmol) was converted to the crude trifluoroacetate salt **16** (pale colored wax, 13.4 mg, quantitative), and used directly for fluorophore coupling. **aHPLC:** (LC: *t*_{R} = 6.51 & 7.80 min); **LCMS** (C18): (LC: *t*_{R} = 7.21 & 8.80 min); **HR-MS** (ESI): calc. for C₄₇H₇₂N₅O₆Si [M+H]⁺: 830.5252, found 830.5241.

### cyclo-[Lys-D-MeTrp-βTyr-(2S,4E,8S)-Htn] trifluoroacetate salt (17)

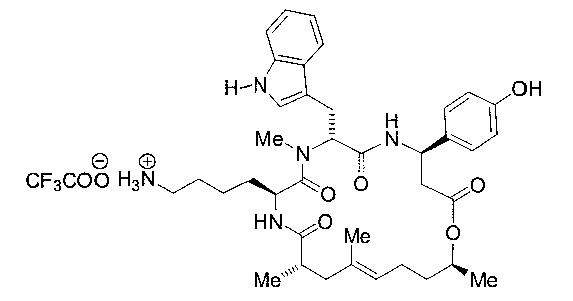

According to the procedure used for the synthesis of **14,** Boc-protected lysine analogue **12** (15.0 mg, 19.4 µmol) was converted to the crude trifluoroacetate salt 17 (pale colored wax, 13.1 mg, quantitative), and used directly for fluorophore coupling. **aHPLC:** (LC: *t*_{R} = 4.52 & 5.08 min); **LCMS** (C18): (LC: *t*_{R} = 5.41 & 6.24 min); **HR-MS** (ESI): calc. for C₃₈H₅₂N₅O₆ [M+H]⁺: 674.3918, found 674.3908.

### cyclo-[Lys-D-MeTrp-βAla-(2S,4E,8S)-Htn] trifluoroacetate salt (18)

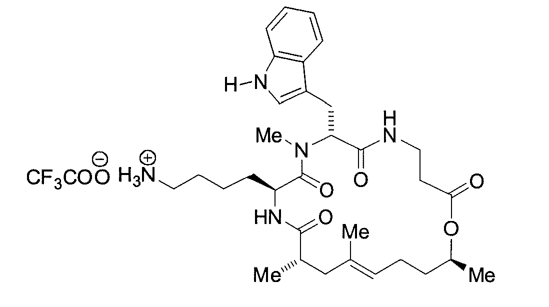

According to the procedure used for the synthesis of **14,** Boc-protected lysine analogue **13** (20.0 mg, 29.3 µmol) was converted to the crude trifluoroacetate salt **18** (pale colored wax, 17.0 mg, quantitative), which was used directly for fluorophore coupling. **aHPLC:** (LC: *t*_{R} = 5.58 min); **LCMS** (C18): (LC: *t*_{R} = 7.84 min); **LR-MS** (ESI): calc. for C₃₂H₄₈N₅O₅ [M+H]⁺: 582.37, found 582.25; calc. for C₃₂H₄₇N₅NaO₅ [M+Na]⁺: 604.35, found 604.37.

### Fluorophore coupling

### BODIPY FL-X-cyclo-[Lys-D-MeTrp-βTyr-(2S,4E,6R, 7R)-Hto] (19D)

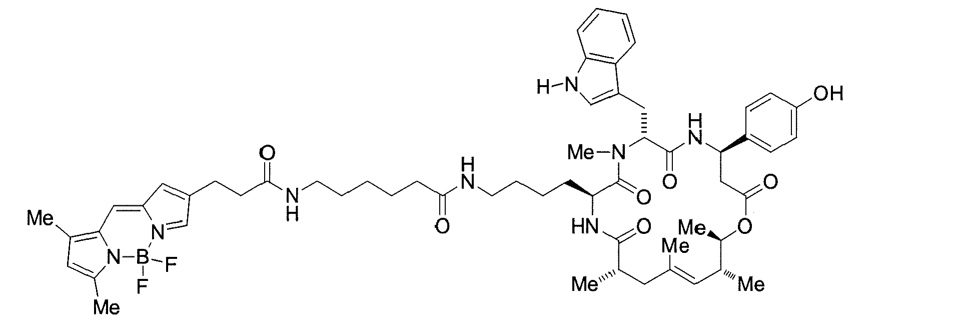

The BODIPY FL-X N-succinimidyl ester dye was pre-weighed (2.5 mg, 4.96 µmol, 1.2 eq.) in an an oven-dried (100 °C, 24 h) 2 mL glass vial fitted with a magnetic stirrer and dissolved in 485 µL dry DMSO. The entire solvent volume was transferred via Gilson micropipette to the crude amine 15 (3.25 mg, 4.13 µmol). *N,N*-diisopropylethylamine (1.0 µL, 5.78 µmol, 1.4 eq.) was added and the reaction immediately covered in aluminium foil, placed in the dark and stirred at rt. Reaction conversion was monitered by LC-MS and aHPLC. After 1 h, no conversion was observed. A further 0.7 eq. base was added after 3 h. After 4 h, complete conversion of the amine was observed with evidence of the desired dye conjugate. Unreacted NHS activated ester BODIPY dye was then quenched through the addition of 0.4 µL ethanolamine. After 6 h, aHPLC and LC-MS analyses clearly showed the complete quenching of the unreacted dye. The reaction mixture was then separated into three equally-sized aliquots, each one diluted to 0.7 mL using HPLC grade MeCN for purification by semi-preparative RP-HPLC (method B) by direct injection to afford **19D** (2.56 mg, 2.41 µmol, 58%) as an orange-colored powder after freeze-drying. **aHPLC:** (LC: *t*_{R} = 6.92 min); **LCMS** (C18): (LC: *t*_{R} = 10.31 min); **¹H NMR** (600 MHz, CD₃CN) δ = 9.96 (br s, 1H; Trp2-NH), 7.59 (d, *J* = 7.8 Hz, 1H), 7.36 (s, 1H), 7.34 (d, *J* = 8.4 Hz, 1H), 7.13 - 7.08 (m), 7.02 (dd, *J* = 7.2, 1.2 Hz, 1H), 7.00 (d, *J* = 4.2 Hz, 1H), 6.98 (d, *J* = 2.4 Hz, 1H), 6.78-6.75 (m, 2H), 6.59 (d, *J* = 7.5 Hz), 6.47-6.45 (m, 2H), 6.31 (d, *J* = 4.0 Hz), 6.21 (s), 5.48 (dd, *J* = 9.0, 7.2 Hz), 5.28 (dd, *J* = 9.0, 3.0 Hz), 4.85 (d, *J* = 9.7 Hz), 4.61 (app q, *J* = 12.8, 6.5 Hz), 4.50 (dq, *J* = 6.6, 5.4, 1H), 3.16 - 3.11 (m), 3.06 - 2.99 (m), 2.93-2.87 (m), 2.88 (s, 3H), 2.61 (dd, *J* = 15.0, 3.6 Hz), 2.57 - 2.50 (m), 2.29 (app. t, *J* = 12.6, 2H), 2.25 (s, 3H), 2.24 - 2.09 (m, signal obscured by solvent), 1.63 (s, 3H), 1.62-1.57 (m, 2H), 1.47-1.43 (m, 2H), 1.32-1.26 (m), 1.22-1.18 (m, 2H), 1.08-1.02 (m), 1.06 (d, *J* = 7.2 Hz), 0.96 (d, J = 6.6 Hz, 3H), 0.90-0.83 (m), 0.88 (d, *J* = 7.2 Hz, 3H), 0.77-0.70 (m) ppm; ¹⁹**F NMR** (188 MHz, CD₃CN): δ = -76.16 (s, C*F*₃COOH), -145.43 (quartet, BODIPY); **HR-MS** (ESI): calc. for C₅₈H₇₆BF₂N₈O₈ [M+H]⁺: 1061.5847, found 1061.5859; calc. for C₅₈H₇₅BF₂N₈NaO₈ [M+Na]⁺: 1083.5667, found 1083.5672.

### Diacetate-protected-5-carboxyfluorescein-cyclo-[Lys-D-MeTrp-βTyr-(2S,4E,6R,7R)-Hto] (19B)

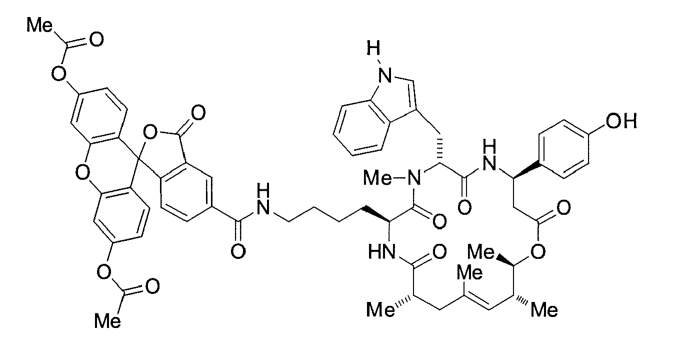

According to the procedure used for the synthesis of compound **19D, 19B** was prepared starting from **15** (505 µg, 0.647 µmol) and using 5-carboxy-fluorescein diacetate N-succinimidyl ester as the dye in Molecular Biology grade DMSO as solvent. Once LC analysis had confirmed complete conversion of the starting material and the formation of **19B,** the reaction was treated with 0.8 eq. ethanolamine to quench unreacted dye label and used directly for cell staining. The concentration of the probe was approximated by back-calculation, assuming 100% formation of the desired compound; **aHPLC:** (LC: *t*_{R} = 8.43 min); **LCMS** (C18): (LC: *t*_{R} = 10.32 min); **HR-MS** (ESI): calc. for C₆₃H₆₆N₅O₁₄ [M+H]⁺: 1116.4606, found 1116.4612; calc. for C₆₃H₆₅N₅NaO₁₄ [M+Na]⁺: 1138.4426, found 1138.4428.

### TAMRA-cyclo-[Lys-D-MeTrp-βTyr-(2S,4E,6R,7R)-Hto] - 5/6-isomers, 1:1 (19C)

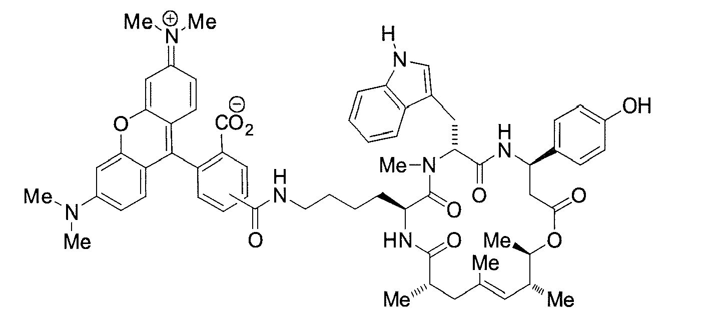

According to the procedure used for the synthesis of compound **19D, 19C** was prepared starting from 15 (400 µg, 0.512 µmol) and using 5/6-carboxytetramethylrhodamine (TAMRA) N-succinimidyl ester as the dye. Semi-preparative RP-HPLC purification (method B) followed by freeze-drying, afforded 19C (510 µg, 0.469 µmol, 92%) as a burgundy-colored powder. **aHPLC:** (LC: *t*_{R} = 6.92 min); **LCMS** (C18): (LC: *t*_{R} = 8.93 min); **HR-MS** (ESI): calc. for C₆₃H₇₂N₇O₁₀ [M+H]⁺: 1086.5341, found 1086.5344.

### 5-carboxyfluorescein-cyclo-[Lys-D-MeTrp-βTyr(OTIPS)-(2S,4E,8S)-Htn] (20A)

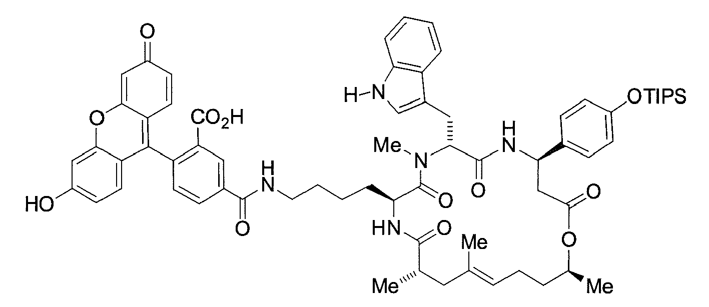

According to the procedure used for the synthesis of compound **19D, 20A** was prepared starting from **16** (7.65 mg, 8.10 µmol) and using 5-carboxy-fluorescein N-succinimidyl ester as the dye. Semi-preparative RP-HPLC purification (method A) followed by freeze-drying, afforded **20A** (2.80 mg, 2.36 µmol, 29%) as an orange-colored powder. **aHPLC:** (LC: *t*_{R} = 10.79 min); **LCMS** (C18): (LC: *t*_{R} = 11.97 min); **HR-MS** (ESI): calc. for C₆₈H₈₂N₅O₁₂Si [M+H]⁺: 1188.5729, found 1188.5725; calc. for C₆₈H₈₁N₅NaO₁₂Si [M+Na]⁺: 1210,5549, found 1210.5544 ; calc. for C₆₈H₈₃N₅O₁₂Si [M+2H]²⁺: 594.7904, found 594.7896.

### Diacetate-protected-5-carboxyfluorescein-cyclo-[Lys-D-MeTrp-βTyr(OTIPS)-(2S,4E,8S)-Htn] (20B)

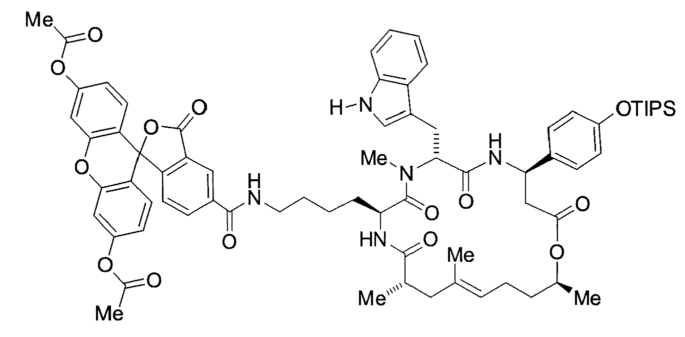

According to the procedure used for the synthesis of compound **19D, 20B** was prepared starting from **16** (4.55 mg, 4.82 µmol) and using 5-carboxy-fluorescein diacetate N-succinimidyl ester as the dye. Semi-preparative RP-HPLC purification (method B) followed by freeze-drying, afforded **20B** (730 µg, 0.57 µmol, 12%) as a white powder. **aHPLC:** (LC: *t*_{R} = 9.04 min); **LCMS** (C18): (LC: *t*_{R} = 10.41 min); **HR-MS** (ESI): calc. for C₇₂H₈₇N₅O₁₄Si [M+2H]²⁺: 636.8010, found 636.8003.

### TAMRA-cyclo-[Lys-D-MeTrp-βTyr(OTIPS)-(2S,4E,8S)-Htn] - 5/6-isomers, 1:1 (20C)

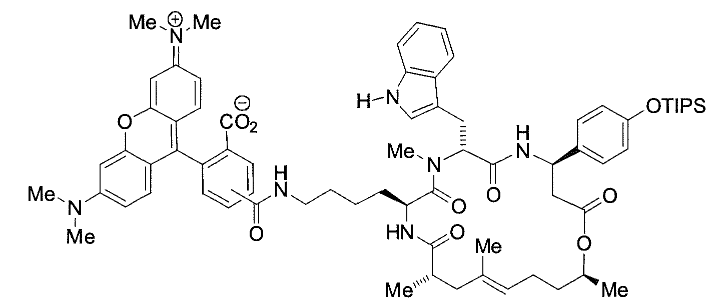

According to the procedure used for the synthesis of compound **19D, 20C** was prepared starting from **16** (4.55 mg, 4.82 µmol) and using 5/6-carboxytetramethylrhodamine (TAMRA) N-succinimidyl ester as the dye. Semi-preparative RP-HPLC purification (method A) followed by freeze-drying, afforded 20C (1.95 mg, 1.57 µmol, 33%) as a burgundy-colored powder. **aHPLC** (LC: t_{R} = 9.20 min); **LCMS** (C18): (LC: t_{R} = 10.91 min); **HR-MS** (ESI): calc. for C₇₂H₉₂N₇O₁₀Si [M+H]⁺: 1242.6675, found 1242.6679.

### 5-carboxyfluorescein-cyclo-[Lys-D-MeTrp-βTyr-(2S,4E,8S)-Htn] (21A)

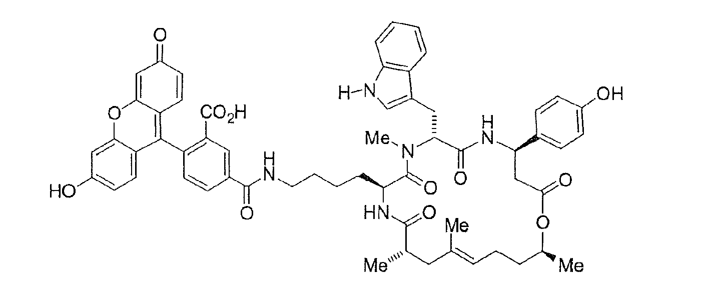

According to the procedure used for the synthesis of compound **19D, 21A** was prepared starting from 17 (4.07 mg, 5.17 µmol) and using 5-carboxy-fluorescein N-succinimidyl ester as the dye. Semi-preparative RP-HPLC purification (method B) followed by freeze-drying, afforded **21A** (1.8 mg, 1.74 µmol, 34%) as an orange powder. **aHPLC:** (LC: *t*_{R} = 7.52 min); **LCMS** (C18): (LC: *t*_{R} = 9.99 min); **HR-MS** (ESI): calc. for Cₛ₉H₆₂N₅O₁₂ [M+H]⁺: 1032.4395, found 1032.4392; calc. for C₅₉H₆₁N₅NaO₁₂ [M+Na]⁺: 1054.4214, found 1054.4213 ; calc. for C₅₉H_{6H}N₅O₁₂ [M+2H]²⁺: 516.7237, found 516.7230.

### Diacetate-protected-5-carboxyfluorescein-cyclo-[Lys-D-MeTrp-βTyr-(2S,4E,8S)-Htn] (21 B)

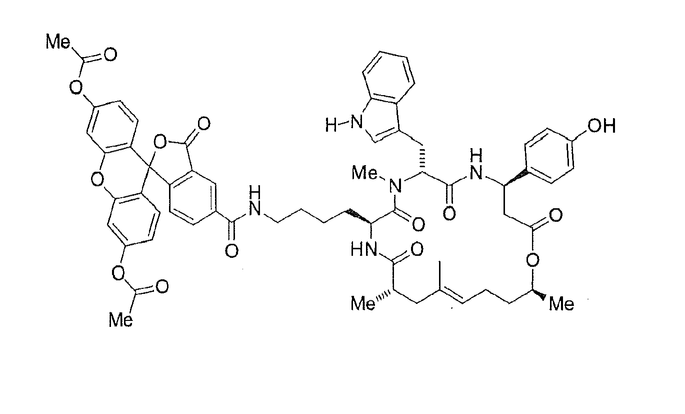

According to the procedure used for the synthesis of compound **19D, 21 B** was prepared starting from 17 (4.17 mg, 5.29 µmol) and using 5-carboxy-fluorescein diacetate N-succinimidyl ester as the dye. Semi-preparative RP-HPLC purification (method B) followed by freeze-drying, afforded **21 B** (780 µg, 0.70 µmol, 13%) as a white powder. **aHPLC:** (LC: *t*_{R} = 8.48 min); **LCMS** (C18): (LC: *t*_{R} = 10.38 min); **HR-MS** (ESI): calc. for C₆₃H₆₆N₅O₁₄ [M+H]⁺: 1116.4606, found 1116.4612; calc. for C₆₃H₆₅N₅NaO₁₄ [M+Na]⁺: 1138.4426, found 1138.4428.

### TAMRA-cyclo-[Lys-D-MeTrp-βTyr-(2S,4E,8S)-Htn] - 5/6-isomers, 1:1 (21 C)

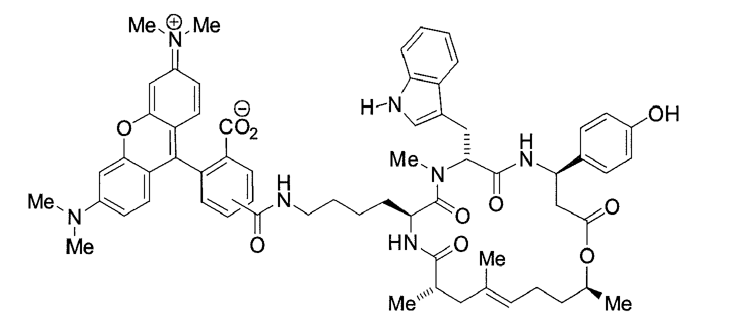

According to the procedure used for the synthesis of compound **19D, 21C** was prepared starting from 17 (403 µg, 0.512 µmol) and using 5/6-carboxytetramethylrhodamine (TAMRA) N-succinimidyl ester as the dye. Semi-preparative RP-HPLC purification (method A) followed by freeze-drying, afforded **21C** (500 µg, 0.46 µmol, 90%) as a burgundy-colored powder. **aHPLC:** (LC: *t*_{R} = 6.98 min); **LCMS** (C18): (LC: *t*_{R} = 8.91 min); **HR-MS** (ESI): calc. for C₆₃H₇₂N₇O₁₀ [M+H]⁺: 1086.5341, found 1086.5345; calc. for C₆₃H₇₁N₇NaO₁₀ [M+Na]⁺: 1108.5160, found 1108.5163.

### TAMRA-cyclo-[Lys-D-MeTrp-βAla-(2S,4E,8S)-Htn] - 5/6-isomers, 1:1 (22C)

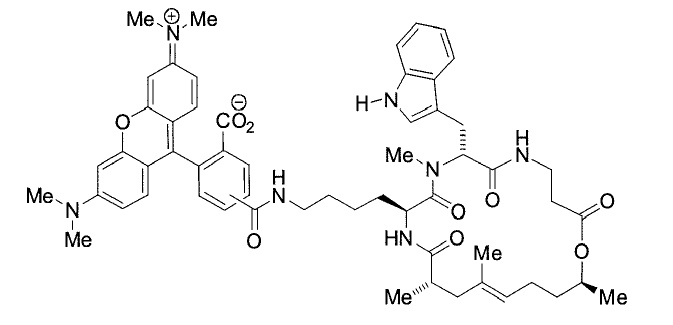

According to the procedure used for the synthesis of compound **19D, 22C** was prepared starting from **18** (20.4 mg, 29.3 µmol) and using 5/6-carboxytetramethylrhodamine (TAMRA) N-succinimidyl ester as the dye. Semi-preparative RP-HPLC purification (method A) followed by freeze-drying, afforded **22C** (18.5 mg, 18.6 µmol, 64%) as a burgundy-colored powder. **aHPLC:** (LC: *t*_{R} = 6.50 & 6.59 min); **LCMS** (C18): (LC: *t*_{R} = 8.78 & 8.89 min); ¹H **NMR** (400 MHz, CD₃OD) δ = 8.78 (d, *J* = 1.6 Hz, 1H), 8.39 (d, *J* = 8.4 Hz, 1H), 8.24 (dd, *J* = 7.6, 1.6 Hz, 1H), 8.19 (dd, *J* = 8.0, 1.6 Hz, 1H), 7.81 (d, *J* = 1.6 Hz, 1H), 7.72-7.65 (deterium exchanging signals), 7.59 (d, J = 7.6 Hz, 1H), 7.54 (d, *J* = 8.0 Hz, 1H), 7.50 (d, *J* = 8.0 Hz, 1H), 7.34 (d, *J* = 8.0 Hz, 1H), 7.28 (d, *J* = 8.0 Hz, 1H), 7.15 - 6.94 (m), 5.63 (dd, *J* = 11.2, 5.2 Hz, 1H), 5.58 (dd, *J* = 11.2, 5.2 Hz, 1H), 5.08 (t, *J* = 7.2 Hz, 1H), 5.03 (t, J = 7.2 Hz, 1H), 4.62 (dd, *J* = 7.6, 5.2 Hz, 1H), 4.62 (dd, *J* = 7.2, 5.2 Hz, 1H), 3.52 - 3.45 (m), 3.43 - 3.09 (m), 3.02 (s, 3H), 2.95 (s, 3H), 2.64 - 2.45 (m), 2.29 - 2.16 (m), 2.06 - 1.82 (m), 1.69 - 1.56 (m), 1.57 (s, 3H), 1.54 (s, 3H), 1.48 - 1.29 (m), 1.22 (d, *J* = 6.4 Hz, 3H), 1.20 (d, *J* = 6.0 Hz, 3H), 1.06 - 0.85 (m), 1.04 (d, *J* = 7.2 Hz, 3H), 0.96 (d, *J* = 7.2 Hz, 3H) ppm; **HR-MS** (ESI): calc. for C₅₇H₆₈N₇O₉ [M+H]⁺: 994.5079, found 994.5072.

### BODIPY FL-X-cyclo-[Lys-D-MeTrp-βTyr-(2S,4E,8S)-Htn] (21 D)

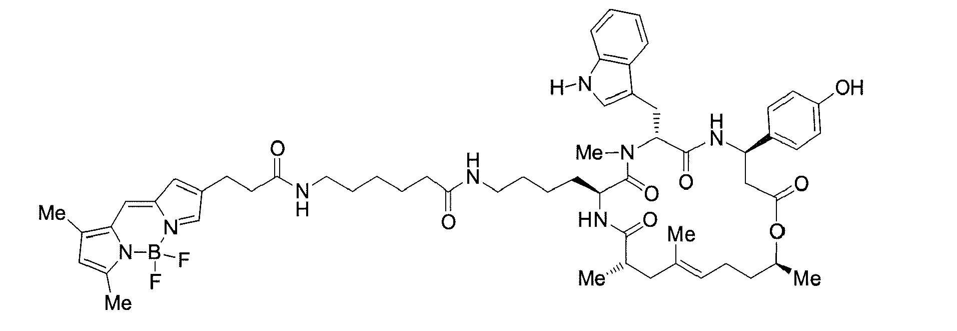

The synthesis of compound 21 D is schematically shown in Scheme 3.

A solution of the crude trifluoroacetate salt **17** (8 mg, 10.15 µmol, 1 eq.), 6-(*tert*-butoxycarbonylamino)hexanoic acid (2.58 mg, 11.17 µmol, 1.1 eq.), HOAt (1.52 mg, 11.17 µmol, 1.1 eq.), HATU (4.25 mg, 11.17 µmol, 1.1 eq.) and *N*-methylmorpholine (2.45 µl, 22.34 mmol, 2.2 eq.) in dry DMSO (3 mL) was stirred at room temperature over night under argon. Upon addition of water (30 mL) the resulting solution was extracted with EtOAc (3 x 10 mL) and the collected organic phase dried (MgSO₄) and concentrated. Column chromatography (CH₂Cl₂/MeOH 98:2 to 95:5) of the crude afforded the 6-(*tert*-butoxycarbonylamino) hexanoic derivative **17a** (5.1 mg, 56%) as a white solid. According to the procedure used for the synthesis of **14, 17a** (4.8 mg, 5.41 µmol, 1 eq.) was converted to the crude trifluoroacetate salt **17b** as a pale colored wax (quantitative, 4.77 mg) which was used directly for fluorophore coupling. The BODIPY carboxylic acid dye was pre-weighed (1 mg, 3.39 µmol, 1.2 eq.) in an oven-dried (100 °C, 24 h) 2 mL glass vial and dissolved in 485 µL dry DMSO. The entire solvent volume was transferred to a solution containing the dry crude trifluoroacetate salt **17b** (2.50 mg, 2.83 µmol, 1 eq.), HATU (1.18 mg, 3.11 µmol, 1.1 eq.), HOAt (0.42 mg, 3.11 µmol, 1.1 eq.) and *N*-methylmorpholine (6.84 µl, 6.23 µmol, 2.2 eq.) and the reaction immediately covered in aluminium foil, placed in the dark and stirred overnight at room temperature. Reaction conversion was monitered by LC-MS and after 14 hours, complete conversion of the amine was observed with evidence of the desired dye conjugate 21 D. The reaction mixture was then separated into three equally-sized aliquots, each one diluted to 0.7 mL using HPLC grade MeCN for purification by semi-preparative HPLC (method B) by direct injection. The collected fractions were combined and freeze-dried to yield the desired dye conjugate **21D** (1.35 mg, 45%) as a orange-colored powder.

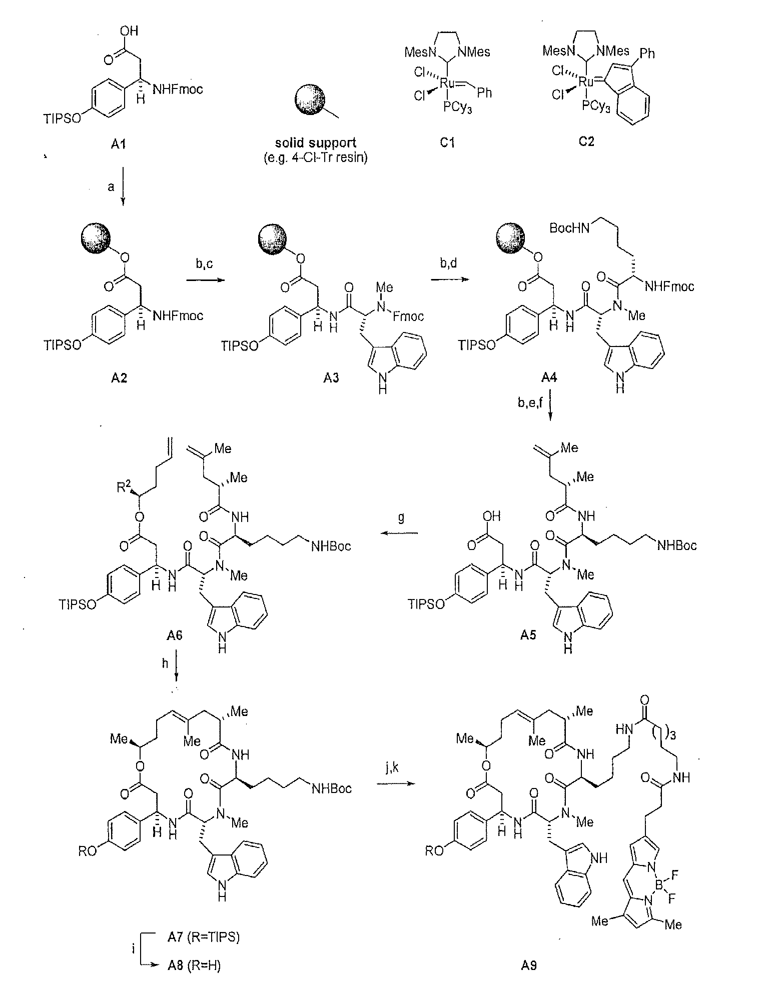

**Scheme 3:** Reagents and conditions: (a) Fmoc-β-phenyltyrosine(OTIPS) **(A1)**, 2-chlorotrityl chloride PS resin, (iPr)₂NEt, CH₂Cl₂, rt, 1 h (loading 0.67 mmol/g); (b) DBU, piperidine, NMP (5 x), rt; (c) D-Fmoc-N_{α}Me-Trp-OH, DIC, HOBt, DMF, rt, 2-3 h; (d) Fmoc-Lys(N_{α}Boc)-OH, HATU, HOAt, (iPr)₂NEt, NMP, rt, 2-3 h; (e) (2S)-2,4-dimethyl-4-pentenoic acid, DIC or HATU, HOBt, (iPr)₂NEt, NMP, rt, 2-3 h; (f) HFIP/CH₂Cl₂ (1 x 20 min, 1 x 10 min); (g) (S)-5-hexen-2-ol, EDC-HCI, (iPr)₂NEt, DMAP, CH₂Cl₂/DMF, rt, 14 h; (h) 10% metathesis catalyst **(C1,** C2, Umicore M5, or M6), CH₂Cl₂, 40°C, Ar purging, 2-6h (TLC or HPLC control), 44-55% yield after prep. HPLC (from A2); (i) TBAF, THF, 30min, 79%; (j) CF₃COOH/CH₂Cl₂ (1:2), 30 min, quant., then 6-(tert-butoxycarbonylamino)hexanoic acid, HATU, HOAt, NMM, DMSO, 18 h, 56%; (k) CF₃COOH/CH₂Cl₂ (1:2), 30 min, quant., then BODIPY carboxylic acid, HATU, HOAt, NMM, DMSO, 14 h, 45% (after prep. HPLC).

**(17a) *R*_{f}** = 0.27 (CH₂Cl₂/MeOH 8:2); **LCMS** (C18): (LC: *t*_{R} = 8.80 min); **¹H NMR** (400 MHz, CDCl₃): δ = 9.91 (s, 1H), 7.61 (d, *J* = 7.8 Hz, 1H), 7.36 (d, *J* = 8.0 Hz, 1H), 7.12 (tt, *J* = 15.1, 7.4 Hz, 4H), 6.96 (s, 1H), 6.78 (t, *J* = 9.7 Hz, 2H), 6.07 (s, 1H), 5.76-5.67 (m, 1H), 5.28-5.17 (m, 1H), 5.01 (t, *J* = 6.2 Hz, 1H), 4.91-4.77 (m, 2H), 3.51 (s, 1H), 3.33 (d, *J* = 8.9 Hz, 2H), 3.25-3.04 (m, 4H), 3.00 (s, 1H), 2.95 (d, *J* = 10.7 Hz, 4H), 2.77 (dd, *J* = 15.2, 3.7 Hz, 1H), 2.68-2.54 (m, 1H), 2.52-2.32 (m, 2H), 2.25 (t, *J* = 7.4 Hz, 2H), 2.19 (s, 1H), 1.95-1.78 (m, 3H), 1.78-1.62 (m, 3H), 1.62-1.49 (m, 4H), 1.46 (m, 6H), 1.38 (dd, *J =* 14.9, 7.1 Hz, 4H), 1.26 (d, *J =* 12.1 Hz, 4H), 1.19-1.07 (m, 7H), 1.01-0.83 (m, 2H), 0.66 (s, 1H) ppm; **HR-MS** (ESI): calc. for C₄₉H₇₀N₆O₉ [M+H]⁺: 887.5238, found 887.5257.

**(21 D) mp:** 187 °C; ***R*_{f}** = 0.23 (EtOAc/cyclohexane 8:2); **aHPLC:** (LC: *t*_{R} = 8.07 min); **LCMS** (C18): (LC: *t*_{R} = 10.33 min); **[α]_{D} ²⁰** = +78.4 *(c* = 0.12 in MeOH); **¹H NMR** (400 MHz, MeOH-*d₄*): δ = 8.34 (d, *J =* 9.3 Hz, 1H), 7.57 (d, *J* = 8.2 Hz, 1H), 7.41 (s, 1H), 7.31 (d, *J* = 7.9 Hz, 1H), 7.12-7.03 (m, 7H), 6.72 (d, *J =* 8.6 Hz, 2H), 6.31 (d, *J =* 3.9 Hz, 1H), 6.20 (s, 1H), 5.62 (t, *J =* 8.2 Hz, 1H), 5.23 (td, *J* = 8.6, 3.2 Hz, 1H), 5.02 (t, 6.3 Hz, 1H), 5.02-4.75 (m, 1H), 3.23-3.19 (m, 12H), 3.08 (s, 3H), 2.97-2.94 (m, 4H), 2.70 (t, *J =* 7.4 Hz, 2H), 2.59 (t, *J* = 7.6 Hz, 3H), 2.50 (s, 3H), 2.27 (s, 3H), 2.14 (t, *J* = 7.6 Hz, 3H), 1.88 (dd, *J* = 13.3, 8.6 Hz, 3H), 1.59 (dd, *J* = 14.1, 7.8 Hz, 4H), 1.54-1.46 (m, 6H), 1.42-1.25 (m, 3H), 1.18 (m, 5H), 1.07 (d, *J* = 7.0 Hz, 3H); ¹⁹**F NMR** (188 MHz, CD₃OD): δ = -77.46 (s, C*F*₃COOH), -145.43 (quartet, BODIPY); **HR-MS** (ESI): calc. for C₅₈H₇₆BF₂N₈O₈ [M+H]⁺: 1061.5847, found 1061.5857; calc. for C₅₈H₇₅BF₂N₈NaO₈ [M+Na]⁺: 1083.5667, found 1083.5671.

### CELL BIOLOGY

### Staining of Neuro-2a and U20S cells

Cell culture and transfection. Neuro-2a cells (DMSZ, Braunschweig, Germany) and U2OS cells (kind gift from Perihan Nalbant, University Duisburg-Essen) were cultured using standard conditions. For microscopic imaging, cells were seeded onto glass-bottom dishes (Mat-Tek Corporation, Ashland, MA) or Lab-Tek chambered coverglass (Nunc GmbH & Co. KG, Langenselbold, Germany). As indicated, one day after plating, cells were either co-transfected with plasmids coding for mCherry-actin (derived by exchange of the fluorescent protein tag in mRFP1-actin for mCherry) and dominant positive Rac1 Q61L (Gary Bokoch, TSRI, La Jolla) for subsequent live cell imaging, or directly processed for staining (Dehmelt et al. Brain Cell Biology 2006, 35, 39-56; Shaner et al. Nat. Biotechnol. 2004, 22, 1567-1572). Transfection of Neuro-2a cells was performed using Fugene-6 reagent (Roche, Mannheim) according to the manufacturer's protocols. Cells were fixed in PBS + 4% formaldehyde for 20 minutes at 37 °C and permeabilized in PBS + 0.25% Triton X-100 for 15 minutes at room temperature, as indicated. Staining of fixed cells with fluorescent jasplakinolide derivatives or Alexa-488 phalloidin (Life Technologies GmbH, Darmstadt), was performed in PBS for 30 minutes at room temperature.

Fluorescence microscopy. lmaging of fixed cells was performed either on the LSM 510 confocal microscope (Carl Zeiss AG, Oberkochen, Germany), or via the wide-field fluorescence mode of a TIRF-M microscope (Olympus, Hamburg, Germany). Live cell imaging was performed one day after transfection, at 35 °C in HEPES-stabilized imaging medium (PAN-Biotech GmbH, Aidenbach, Germany) using total internal reflection fluorescence microscopy (TIRF-M). For TIRF-M, an Olympus lX-81 microscope, equipped with a PlanAPO60xOil TIRFM objective (NA=1.45) and 1.6 optovar was used. A triple bandpass dichroic mirror (U-M3TlR405/488/561, Olympus) was combined with Semrock Brightline emision filters (HC 520/35 and HC 629/53, AHF Analysentechnik, Tübingen, Germany), the 488nm line of a 400mW Argon ion laser (model # 543-A-A03, Melles Griot, Bensheim, Germany) and a 561nm, 25mW CellR diode laser (Olympus). For detection, an EMCCD camera (C9100-13, Hamamatsu, Herrsching, Germany) was used at maximal gain. All microscope components were controlled by the CellR software (Olympus). During live cell imaging, all additions of jasplakinolide derivatives were performed on the microscopy stage. Kymograph analysis was performed via the Multiple Kymograph plugin (Rietdorf and Seitz, EMBL, Heidelberg) for lmageJ (NIH, Bethesda).

Staining of HeLa cells. HeLa cells were seeded 20-48 hours before measurement in round 2 ml-dishes using DMEM +10% FBS, 1% NEAA, 1% glutamine as medium. The cell-dishes were incubated in an incubation chamber at 37 °C and 5% CO2. For measuring, the medium was exchanged against 1 x PBS (pre-warmed to 37°C). The cell dish was placed in the microscope tray held at 37 °C and then the compounds were supplemented to 1 µM final concentration. Time points were taken every 5-15 minutes.

Staining of BSC-1 cells. BSC-1 (African green monkey) cells were cultured in 96-well plates at a density of approx. 2000 cells per well for 24 hours using standard protocols. Compounds dissolved in DMSO were introduced to a final concentration of ca. 200 nM, 500 nM, 1 µM, 5 µM, 10 µM and 30 µM. Jasplakinolide (Invitrogen) was employed as a positive control at a final concentration of 100 nM. The cells were incubated for 16 hours with the synthesized compounds, jasplakinolide or an equivalent volume of DMSO. After treatment the cells were fixed with 4% formaldehyde (80 mM PIPES, pH 6.8, 1 mM MgCl₂, 1 mM EGTA, 0.1% Triton X-100) and stained for actin and chromatin with TRITC labeled phalloidin (Sigma) and DAPI (Sigma), respectively. lmages were acquired on an automated microscope (Zeiss 200M) at 40x magnification.

### Fluorescent labeling of F-actin in fixed cells

Analysis of staining efficiency revealed that fluorescently-labeled compounds **19** and **21 -** selectively and efficiently stain F-actin in several mammalian cell lines after fixation (Figure 3). In N2a, BSC-1, and HeLa cells, characteristic and intense labeling of actin-rich filipodia, lamellipodia and actin bundles was observed after incubation with **19C, 21 B,** and 21C (see Figure 3). Next, more extensive confocal microscopy studies of stained human osteosarcoma U20S cells were performed on a subset of probes (Figure 4). TAMRA-labeled analogs **19C** and **21C** visualized typical actin structures in maximum projections of Z-stacks. While cell staining at 1 µM **19C** was weaker without permeabilization, the staining efficiency of **21C** at the same concentration was independent of permeabilization. The overall labeling patterns were very similar when fixed and permeabilized U2OS cells were simultaneously co-stained with Alexa-488 phalloidin and 1 µM **19C** (Figure 4b). Direct comparison of individual Z-sections near (bottom) and far (top) from the glass substrate in the two regions of interest revealed that Alexa-488 phalloidin and 19C both labelled the same actin structures, including filopodia, lamelipodia, transversal arcs, dorsal stress fibers, ventral stress fibres, and dorsal ruffles (Figure 4c). No staining preference among these structures was observed for either of the two probes.

### Selective labeling of long-lived F-actin structures in living cells

The observation that non-permeabilized cells were efficiently labeled by some of the components prompted one to investigate living cells. After transfection and expression of a dominant-positive mutant of the small GTPase Rac1, Neuro-2a neuroblastoma (N2a) cells (Klebeet al. J. Ce/l Biol. 1969, 43, 69A) form extensive, dynamic lamellipodia and filopodia, as well as less dynamic, long-lived stress-fibers and dynamically intermediate actin arc structures (Figure 5a). Therefore, these cells seemed optimally suited to evaluate the interaction of the fluorescent probes according to the invention with F-actin structures characterized by distinct dynamic turnover rates.

Although the TAMRA-labeled analog **21C** was able to efficiently stain non-permeabilized U20S cells, only weak staining of living N2a cells by the same probe could be detected following a brief exposure and washout. The BODIPY-labeled analogs **19D** and **21D** were capable of efficient and selective labeling of F-actin in living cells (see Figure 3 and Figure 5, respectively). To investigate the full capability of these probes in live cells, subsequent experiments were performed using the more synthetically accessible **21D.** Data from TIRF (total internal reflection fluorescence) microscopy imaging (Axelrod, D. J. Cell Biol. 1981, 89, 141-145) clearly showed that long- or intermediate-lived structures, such as stress fibers or actin arcs (Figure 5a, insets) were strongly labeled by **21 D.** However, highly dynamic structures undergoing rapid turnover, such as lamellipodia or filopodia, were not labeled by **21 D,** although they were clearly indicated by overexpressed mCherry-actin.

Importantly, kymograph analysis of living cells labeled with probe **21 D** showed that F-actin remained highly dynamic in lamellipodia (Figure 5b), despite the presence of the probe according to the invention. This stands in stark contrast to the natural product **4,** which is widely documented to induce a rapid collapse of dynamic F-actin structures. In control experiments, unlabeled compound **12** (10 µM) seems to influence actin dynamics shortly after application and induced a collapse of lamellipodia, while **21 D** did not inhibit actin dynamics (Figure 5C), suggesting that the fluorescent label in **21 D** reduces the compound's actin stabilizing activity. Indeed, **21 D** did not provoke any noticeable change in actin dynamics or cell morphology at the high test concentration of 10 µM.

The above results show that the compounds according to the present invention are suitable as probes for imaging actin structures, particularly in living cells, without significantly influencing actin dynamics.

The subject-matter of the present invention also comprises following items:
1. A compound of Formula (I) 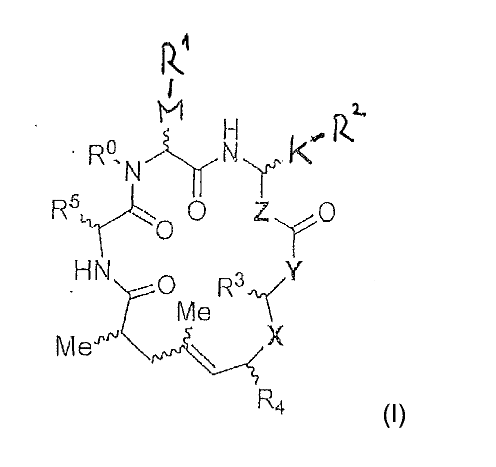 wherein
   R° is hydrogen, hydroxy, substituted or unsubstituted C₁₋₆-alkyl, substituted or unsubstituted hydroxy-C₁₋₆-alkyl, substituted or unsubstituted hydroxy-C₃₋₈-cycloalkyl or a substituted or unsubstituted 3-14 membered carbocyclic ring comprising 0-2 heteroatoms selected from the group consisting of N, O or S,
   M is a bond or -(CH₂)ₙ- with n being an integer of 1-4, preferably -(CH₂)-,
   K is a bond or -(CH₂)ₙ-, preferably a bond,
   R¹ and R² independently are hydrogen, halogen, a 3-14 membered carbocylic ring comprising 0-2 heteroatoms selected from the group consisting of N, O or S, optionally substituted by at least one of halogen, -R⁶, -OR⁶, -SR⁶, -N(R⁶)₂, -NO₂, wherein R⁶ independently is hydrogen, substituted or unsubstituted C₁₋₆-alkyl, substituted or unsubstituted C₃₋₈-cycloalkyl or a protective group,
   R³, R⁴ independently are hydrogen, unsubstituted or substituted C₁-C₆-alkyl,
   R⁵ is -L-CG, wherein -L- is a spacer or a bond and -CG is an optionally protected coupling group,
   X and Z independently are a bond or -CHR⁷-, wherein R⁷ is hydrogen or unsubstituted or substituted C₁-C₆-alkyl,
   Y is -CR⁹R¹⁰-, -NR⁹-, -N(OR⁹)-, -N(NR⁹R¹⁰)-, -NR⁹NR¹⁰-, or -O-, particularly -O-, and
   R⁹, R¹⁰ independently are hydrogen, hydroxy, amino, unsubstituted or substituted C₁₋₆-alkyl, or unsubstituted or substituted C₃₋₈-cycloalkyl.
2. The compound according to item 1,
   wherein the C=C-double bond in formula (I) is E- or Z- configured, particularly E-configured.
3. The compound according to item 1 or 2, having the formula (II) 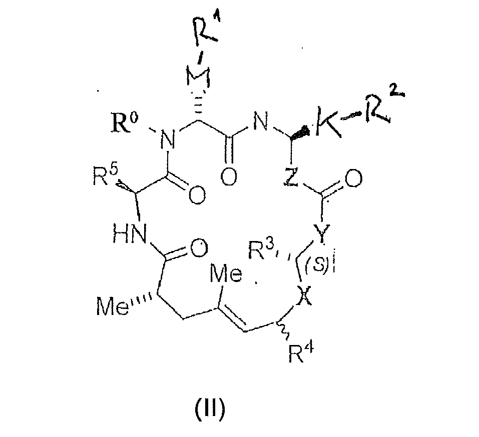
4. The compound according to any of items 1-3, wherein R° is substituted or unsubstituted C₁-C₆-alkyl, particularly methyl, ethyl, propyl, more preferably methyl.
5. The compound according to any of items 1-4, wherein R¹ is substituted or unsubstituted 3-14 membered aromatic or heteroaromatic ring comprising 0-2 heteroatoms selected from the group consisting of N, O or S.
6. The compound according to item 5, wherein R¹ is indolyl, preferably 3-indolyl, optionally substituted in 1-position.
7. The compound according to any of items 1-6, wherein R² is hydrogen.
8. The compound according to any one of items 1-6, wherein R² is a substituted or unsubstituted 3-14-membered aromatic ring, such as phenyl or naphthyl, preferably phenyl.
9. The compound according to any of items 1-6 and 8, wherein the carbocyclic ring of R² is substituted by at least one -OR⁶.
10. The compound according to item 9, wherein R⁶ is hydrogen or TIPS, preferably hydrogen.
11. The compound according to any one of items 8-10, wherein the aromatic ring is at least substituted in para-position.
12. The compound according to any one of items 1-11, wherein R³ is methyl.
13. The compound according to any one of items 1-12, wherein R⁴ is hydrogen.
14. The compound according to any one of items 1-12, wherein R⁴ is methyl.
15. The compound according to any one of items 1-14, wherein X or/and Z is -CHR⁷-.
16. The compound according to item 15, wherein R⁷ is hydrogen.
17. The compound according to any one of items 1-14, wherein X is a bond.
18. The compound according to any one of items 1-17, wherein L is selected from the group consisting of -(CH₂)m⁻, -(CH₂CH₂O)ₘ-, -(CH₂)ₘ-NHC(O)-(CH₂)ₘ-, -(CH₂)ₘ-OC(O)-(CH₂)ₘ-, particularly -(CH₂)ₘ- or -(CH₂)ₘ-NHC(O)-(CH₂)ₘ-, wherein m independently is an integer of 0-6, particularly 1-6, particularly 2-4, more particularly 4 or 5.
19. The compound according to any one of items 1-18, wherein the coupling group CG is -NHR⁸, -OR⁸, particularly -NHR⁸, wherein R⁸ is preferably hydrogen, or a protective group, such as Boc, Fmoc, Cbz, Ts, Alloc, preferably Boc.
20. The compound according to any one of items 1-19, wherein R⁵ is -(CH₂)₄-NHBoc.
21. The compound according to item 19, wherein R⁸ is a diagnostic and/or therapeutic agent.
22. The compound according to item 21, wherein the diagnostic agent and/or therapeutic agent is coupled at the coupling group via an amide, ester, ether, thioether, amine, carbonate, carbamate, urea, or semicarbazide linkage, preferably via an amide linkage.
23. The compound according to item 21 or 22, wherein the diagnostic agent is selected from the group consisting of a radionuclide, dye, particularly a fluorescent dye, a spin label, a metal chelating agent, chromogens or cofactors such as luziferines, a protein binding group such as biotin, a protein reactive group such as a Michael acceptor, a photoreactive group such as benzophenone, or an antigenic moiety, and the therapeutic agent is selected from a toxin, a drug, a signaling molecule or hormone, or peptide.
24. The compound according to item 23, wherein the fluorescent dye is a fluorescein, diacetylated fluorescein, TAMRA, BODIPY, rhodamine, coumarine, cyanine or derivatives thereof, particularly BODIPY or TAMRA, more particularly BODIPY.
25. A process for the manufacture of a compound according to any of items 1-24 comprising the steps:
   (i) providing a compound according to Formula (III) 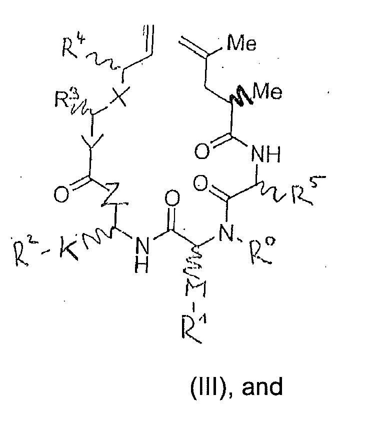
   (ii) converting the compound of Formula (III) to a compound according to Formula (I) using a metathesis catalyst.
26. Use of a compound according to any of items 1-24 for targeting, particularly imaging actin, particularly actin filaments.
27. Use according to item 26, wherein the imaging of actin is in live cells, histological sections or bulk tissue.
28. Use according to item 26 or 27, wherein the actin dynamics are maintained.
29. Use according to any of items 26-28 for imaging static actin filaments.
30. Use according to item 29, wherein the static actin filaments are selectively imaged against dynamic actin populations.
31. Use according to item 30 wherein the actin populations are F-actin and monomeric G-actin populations.

## Claims

1. A compound of Formula (I) 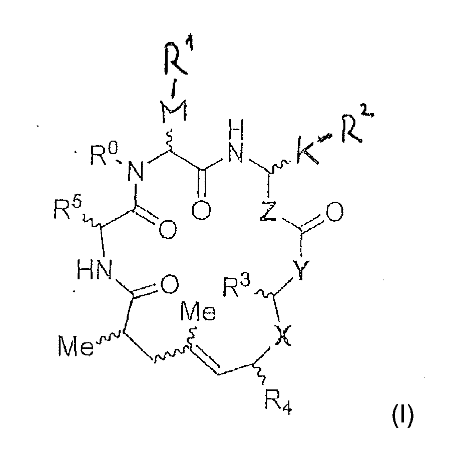 wherein
R° is hydrogen, hydroxy, substituted or unsubstituted C₁₋₆-alkyl, substituted or unsubstituted hydroxy-C₁₋₆-alkyl, substituted or unsubstituted hydroxy-C₃₋₈-cycloalkyl or a substituted or unsubstituted 3-14 membered carbocyclic ring comprising 0-2 heteroatoms selected from the group consisting of N, O or S,
M is a bond or -(CH₂)ₙ- with n being an integer of 1-4, preferably -(CH₂)-,
K is a bond or -(CH₂)ₙ-, preferably a bond,
R¹ and R² independently are hydrogen, halogen, a 3-14 membered carbocylic ring comprising 0-2 heteroatoms selected from the group consisting of N, O or S, optionally substituted by at least one of halogen, -R⁶, -OR⁶, -SR⁶, -N(R⁶)₂, -NO₂ wherein R⁶ independently is hydrogen, substituted or unsubstituted C₁₋₆-alkyl, substituted or unsubstituted C₃₋₈-cycloalkyl or a protective group,
R³, R⁴ independently are hydrogen, unsubstituted or substituted C₁-C₆-alkyl,
R⁵ is -L-CG, wherein -L- is a spacer or a bond and -CG is an optionally protected coupling group,
X and Z independently are a bond or -CHR⁷-, wherein R⁷ is hydrogen or unsubstituted or substituted C₁-C₆-alkyl,
Y is -CR⁹R¹⁰-, -NR⁹-, -N(OR⁹)-, -N(NR⁹R¹⁰)-, -NR⁹NR¹⁰-, or -O-, particularly -O-, and
R⁹, R¹⁰ independently are hydrogen, hydroxy, amino, unsubstituted or substituted C₁₋₆-alkyl, or unsubstituted or substituted C₃₋₈-cycloalkyl, wherein the C=C-double bond in formula (I) is E- or Z- configured, particularly E-configured.

2. The compound according to claim 1, having the formula (II)

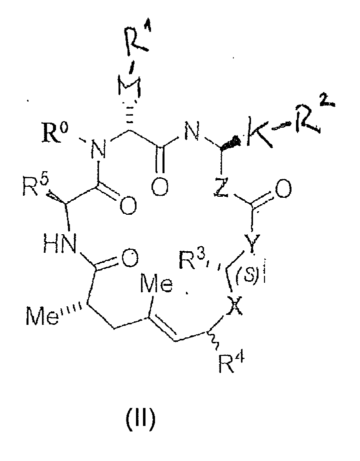

3. The compound according to any of claims 1 or 2, wherein R° is substituted or unsubstituted C₁-C₆-alkyl, particularly methyl, ethyl, propyl, more preferably methyl.

4. The compound according to any of claims 1-3, wherein R¹ is substituted or unsubstituted 3-14 membered aromatic or heteroaromatic ring comprising 0-2 heteroatoms selected from the group consisting of N, O or S, preferably indolyl, more preferably 3-indolyl, optionally substituted in 1-position.

5. The compound according to any of claims 1-4, wherein R² is hydrogen or a substituted or unsubstituted 3-14-membered aromatic ring, such as phenyl or naphthyl, preferably phenyl, wherein the carbocyclic ring of R² is preferably substituted by at least one -OR⁶, with R⁶ being preferably hydrogen or TIPS.

6. The compound according to any one of claims 1-5, wherein R³ is methyl.

7. The compound according to any one of claims 1-6, wherein R⁴ is hydrogen or methyl.

8. The compound according to any one of claims 1-7, wherein X or/and Z is -CHR⁷-, wherein R⁷ is preferably hydrogen.

9. The compound according to any one of claims 1-7, wherein X is a bond.

10. The compound according to any one of claims 1-9, wherein L is selected from the group consisting of -(CH₂)ₘ-, -(CH₂CH₂O)ₘ-, -(CH₂)ₘ-NHC(O)-(CH₂)ₘ-, -(CH₂)ₘ-OC(O)-(CH₂)ₘ-, particularly -(CH₂)ₘ- or -(CH₂)ₘ-NHC(O)-(CH₂)ₘ-, wherein m independently is an integer of 0-6, particularly 1-6, particularly 2-4, more particularly 4 or 5.

11. The compound according to any one of claims 1-10, wherein the coupling group CG is -NHR⁸, -OR⁸, particularly -NHR⁸, wherein R⁸ is preferably hydrogen, or a protective group, such as Boc, Fmoc, Cbz, Ts, Alloc, preferably Boc.

12. The compound according to claim 11, wherein R⁸ is a diagnostic and/or a therapeutic agent, such as a radionuclide, dye, particularly a fluorescent dye, such as fluorescein, diacetylated fluorescein, TAMRA, BODIPY, rhodamine, coumarine, cyanine or derivatives thereof, particularly BODIPY or TAMRA, more particularly BODIPY, a spin label,
a metal chelating agent, chromogens or cofactors such as luziferines, a protein binding group such as biotin, a protein reactive group such as a Michael acceptor, a photoreactive group such as benzophenone, or an antigenic moiety, and the therapeutic agent is selected from a toxin, a drug, a signaling molecule or hormone, or peptide.

13. The compound according to claim 12, wherein the diagnostic agent and/or therapeutic agent is coupled at the coupling group via an amide, ester, ether, thioether, amine, carbonate, carbamate, urea, or semicarbazide linkage, preferably via an amide linkage.

14. A process for the manufacture of a compound according to any of claims 1-13 comprising the steps:
(i) providing a compound according to Formula (III) 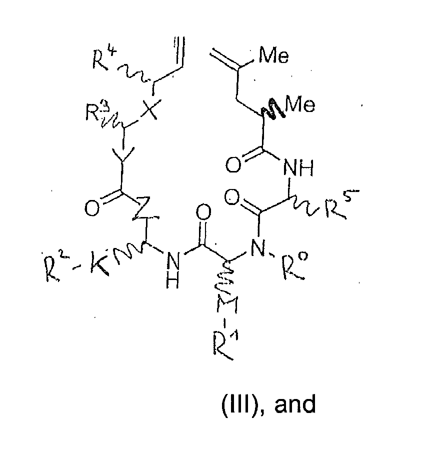
(ii) converting the compound of Formula (III) to a compound according to Formula (I) using a metathesis catalyst.

15. Use of a compound according to any of claims 1-13 for targeting, particularly imaging actin, particularly actin filaments, preferably in live cells, histological sections or bulk tissue, wherein the actin dynamics are preferably maintained, preferably for imaging static actin filaments.
